# EUROPEAN PATENT APPLICATION

(11) **EP 4 756 816 A1**
(43) Date of publication of application: **10.06.2026**
(21) Application number: 25221126.3
(22) Date of filing: 05.12.2025
(51) Int. Cl.: G16C 20/20, G16C 20/70

(54) **ANNOTATION OF TARGET SPECTROMETRY DATA**

(30) Priority: 05.12.2024 US 202418969759
(71) Applicant: HighChem s.r.o., 821 09 Bratislava (SK)
(72) Inventor: RAAB, Michal, Bratislava (SK)
(74) Representative: Boult Wade Tennant LLP

(57) **Abstract**

Embodiments described herein relate to target sample data annotation. A system can comprise a memory that stores, and a processor that executes, computer executable components. The computer executable components can comprise an encoding component that encodes target molecular data for a target sample into a vectorized format, resulting in encoded target molecular data, and a matching component that generates a predicted match of the encoded target molecular data to known neutral loss data for a known sample, the known neutral loss data defining a delta mass-to-charge ratio between spectral values of known spectral data corresponding to the known sample.

## Description

### BACKGROUND

Identification and/or comparison of aspects of spectrometry data from one or more chemical structure measurement devices, datastores, data libraries, etc. can be a complicated and time-intensive process. One or more variables of different data types, data formats, different systems employed to generate the data, different samples, different times and/or lifecycles of execution, different user entity, etc. can affect ability to accurately and/or efficiently conduct the identification and/or comparison of compounds, fragmentation ions and/or neutral losses. Indeed, such one or more variables can cause false positive and/or false negative identification, low probability identification, lack of accurate comparison, etc. In one or more other cases, execution of an identification and/or comparison can be wholly inefficient, based on manual examination of a large plurality of standard spectrometry data, structural data, molecular data, etc.

### SUMMARY

The following presents a summary to provide a basic understanding of one or more example embodiments described herein. This summary is not intended to identify key or critical elements, and/or to delineate scope of particular embodiments or scope of claims. Its sole purpose is to present concepts in a simplified form as a prelude to the more detailed description that is presented later. In one or more example embodiments, systems, computer-implemented methods, apparatuses and/or computer program products described herein can provide a plug-and-play process for using data generated by a measurement instrument (also herein referred to as a measurement device) and/or obtained from a datastore/data library to aid in annotating unknown or target data with structural feature identification, neutral loss identification, fragmentation ion identification and/or other spectral features in a time efficient and automatic manner.

In accordance with an embodiment, a system can comprise a memory that stores computer executable components, and a processor that executes the computer executable components. The computer executable components can comprise an encoding component that encodes target molecular data for a target sample into a vectorized format, resulting in encoded target molecular data, and a matching component that generates a predicted match of the encoded target molecular data to known neutral loss data for a known sample, the known neutral loss data defining a delta mass-to-charge ratio between spectral values of known spectral data corresponding to the known sample.

In accordance with another embodiment, a computer-implemented method can comprise encoding, by a system operatively coupled to a processor, target molecular data for a target sample into a vectorized format, resulting in encoded target molecular data, and generating, by the system, a predicted match of the encoded target molecular data to known neutral loss data for a known sample, the known neutral loss data defining a delta mass-to-charge ratio between spectral values of known spectral data corresponding to the known sample.

In accordance with another embodiment, a computer program product, facilitating a process for target sample annotation, can comprise a computer readable storage medium having program instructions embodied therewith. The program instructions can be executable by a processor to cause the processor to encode, by the processor, target molecular data for a target sample into a vectorized format, resulting in encoded target molecular data, and generate, by the processor, a predicted match of the encoded target molecular data to known neutral loss data for a known sample, the known neutral loss data defining a delta mass-to-charge ratio between spectral values of known spectral data corresponding to the known sample.

The one or more example embodiments described herein can be implemented within, in connection with and/or coupled to a chemical structure measurement device, such as a scientific measurement device, such as a spectrometry device.

The one or more example embodiments disclosed herein can be applied on a plug-and-play basis to a measurement device, plural measurement devices, a same measurement device using plural exchangeable components (e.g., columns), etc. for comparison of output data relative to unknown, known and/or standard data. The frameworks described herein can be performed in a time efficient and at least partially automatic manner, thereby reducing labor processes, increasing accuracy, and providing automatic reasoning for predictions made. In one or more cases, identification data obtained from use of the one or more example embodiments can be employed to construct a database of known molecular, neutral loss, and/or spectral data.

The one or more example embodiments described herein can be employed to generate inferences and/or neutral loss data corresponding to a target sample that would not otherwise be available by merely comparing molecular data and/or neutral loss data for the target sample to a library of known molecular data and/or known neutral loss data. That is, based on spectral data defining a spectrum, one or more neutral losses can be exhibited, while one or more other neutral losses can be non-exhibited. That is, such non-exhibited neutral losses can fail to appear during fragmentation such as due to a chemical structure (e.g., chemical bond type), chemical property, fragmentation energy not being reached, etc. Put another way, the non-exhibited neutral loss can correspond to an ion that has not fragmented from a target sample due to a same and/or different reason (e.g., chemical structure, chemical property, fragmentation energy). As an example, a non-exhibited neutral loss can be a neutral loss than can require a higher energy applied to the sample to cause the neutral loss, such as to break a particular chemical bond, than has been yet applied.

The one or more example embodiments described herein can be employed to leverage information related to one or more compounds different from a target compound for which annotation is desired. For example, a prediction regarding identification of a neutral loss (exhibited or not exhibited in spectral data), a prediction regarding identification of an ion based on a chemical structure (e.g., chemical bond type), and/or a prediction regarding identification of a target compound, without being limited thereto, can be made based on molecular structure data, neutral loss data and/or spectral data, without being limited thereto, that corresponds to a known compound different from the target compound. Such known compound can be of a same family, chemical category type, etc., and/or can have one or more structural features, ions and/or neutral losses in common with the target compound, for example.

The one or more example embodiments described herein can be employed to employ encoding of data in a universal format employable for search, comparison, identification and/or annotation of molecular data, spectral data and/or neutral loss data relating to a plurality of compounds. That is, by use of a universal format, such as an encoded or vectorized format, to be discussed below, comparison can be made where, previously, in existing frameworks, such search, comparison, identification and/or annotation is not possible. In one non-limiting example, molecular structure data, neutral loss data and/or spectral data, without being limited thereto, can all or partially be encoded in a same vectorized format for a same compound, such as in one or more specified data aspects (e.g., comprising data and/or metadata in any suitable form), thereby allowing for efficient comparison with other such data aspects, and/or with other data (e.g., target compound data) also in the vectorized format.

The one or more example embodiments described herein can employ one or more such data aspects, e.g., comprising molecular structure data, neutral loss data and/or spectral data, to compare ion identifications, compound molecular structures, spectral peak values, neutral loss values (e.g., gaps between spectral peaks), etc. of one or more target compounds and/or known compounds. Such comparison can be employed to annotate unknown and/or target compound data and/or to generate a data library of data aspects. Such comparison can be accomplished employing a database of hundreds, thousands, tens of thousands, or more sets of data aspects, without being limited thereto.

Moreover, based on the comparison, a more comprehensive understanding of the target spectral data can be obtained, as compared to existing frameworks. For example, one or more structural and/or neutral loss characteristics can be predicted by use of a model, such as an artificial intelligence (AI) model or machine learning (ML) model employing the database and having learned correspondences among molecular structure data, neutral loss data and/or spectral data for the data aspects comprised by the database. One or more resultant identified peaks, characteristics, ions, neutral losses, etc., relating to a known or unknown compound can be predicted, with one or more outputs being predicted per such result, such as in a ranked and/or weighted format. In one or more cases, ranked and/or weighted data can be accompanied by and/or provided separately from one or more correspondence-based (e.g., correspondences among the molecular structure data, neutral loss data and/or spectral data) reasons for the ranked and/or weighted data. This can allow for an understanding of target molecular structure data, neutral loss data and/or spectral data and its causes and/or the reasoning behind any one or more identifications provided by the model.

### BRIEF DESCRIPTION OF THE DRAWINGS

Embodiments will be readily understood by the following detailed description in conjunction with the accompanying drawings. To facilitate this description, like reference numerals designate like structural elements. Embodiments are illustrated by way of example, not by way of limitation, in the figures of the accompanying drawings.
FIG. 1 illustrates a block diagram of an example, non-limiting system that can facilitate a process for target data annotation, in accordance with one or more example embodiments described herein.
FIG. 2 illustrates a block diagram of another example, non-limiting system that can facilitate a process for target data annotation, in accordance with one or more example embodiments described herein.
FIG. 3 illustrates a flow diagram of training processes for a model that can be performed by the non-limiting system of FIG. 2, in accordance with one or more example embodiments described herein.
FIG. 4 illustrates a flow diagram of one or more training processes based on employing neutral loss data as base data, as can be performed by the non-limiting system of FIG. 2, in accordance with one or more example embodiments described herein.
FIG. 5 illustrates a flow diagram of one or more training processes based on employing molecular data as base data, as can be performed by the non-limiting system of FIG. 2, in accordance with one or more example embodiments described herein.
FIG. 6 illustrates a flow diagram of execution processes that can be performed by the non-limiting system of FIG. 2, in accordance with one or more example embodiments described herein.
FIG. 7 illustrates another flow diagram of execution processes that can be performed by the non-limiting system of FIG. 2, in accordance with one or more example embodiments described herein.
FIG. 8 illustrates a flow diagram of one or more processes that can be performed by the non-limiting system of FIG. 1, in accordance with one or more example embodiments described herein.
FIG. 9 illustrates a flow diagram of one or more processes that can be performed by the non-limiting system of FIG. 2, in accordance with one or more example embodiments described herein.
FIG. 10 illustrates a continuation of the flow diagram of FIG. 9 of one or more processes that can be performed by the non-limiting system of FIG. 2, in accordance with one or more example embodiments described herein.
FIG. 11 illustrates another flow diagram of one or more processes that can be performed by the non-limiting system of FIG. 2, in accordance with one or more example embodiments described herein.
FIG. 12 illustrates a continuation of the flow diagram of FIG. 11 of one or more processes that can be performed by the non-limiting system of FIG. 2, in accordance with one or more example embodiments described herein.
FIG. 13 illustrates a block diagram of an example operating environment into which embodiments of the subject matter described herein can be incorporated.
FIG. 14 illustrates an example schematic block diagram of a computing environment with which the subject matter described herein can interact and/or be implemented at least in part.

### DETAILED DESCRIPTION

The following detailed description is merely illustrative and is not intended to limit embodiments and/or application or utilization of embodiments. Furthermore, there is no intention to be bound by any expressed or implied information presented in the preceding Summary section, or in the Detailed Description section.

Turning first to the subject of chemical structure measurement devices generally, such measurement devices can comprise, but are not limited to spectrometry devices, chromatography devices, etc. Output from such devices can be measurement data defining intensities, mass-to-charge ratios, ion conductivities etc. of analytes, compounds and/or ions analyzed, eluted and/or fragmented during analysis, without being limited thereto. One such type of measurement data can be spectrometry data (also referred to herein as spectral data) resulting from operation of a spectrometry device. To allow for comparison of such spectral data from different analysis runs, plural compounds and/or plural devices, and/or against one or more known and/or standardized datasets, it can be advantageous to employ a baseline for such comparison. Such baseline can comprise use of known/control/standard spectrometry, molecular and/or neutral loss datasets. However, this can be tedious, inefficient, and time consuming, in view of comparison to hundreds, thousands or more analyte standard chromatography datasets.

Further, simple comparison will generally fail to resolve accurate ion fragment identification and/or neutral loss identification. Further still, such existing frameworks cannot provide one or more resultant predicted peaks, characteristics, ions, neutral losses, etc., with one or more outputs being predicted per such result in a ranked and/or weighted format. Moreover, existing frameworks are limited to comparison of datasets having a same format, and thus, when having different formats, datasets are incompatible for such comparison. Accordingly, in connection therewith, use of existing frameworks for dataset annotation can result in failure to identify ions fragmented from a sample and/or neutral losses associated therewith, false positive identification, false negative identification and/or two or more identifications from which a more accurate output cannot be determined.

To account for one or more of these deficiencies, the one or more embodiments described herein can provide a process for employing learned correspondences amongst molecular structural data, neutral loss data and/or spectral data, without being limited thereto, to predict one or more molecular structural features, neutral losses and/or fragmentation ion identifications relative to a set of chemical data defining a target sample.

As used herein, molecular structural data can refer to data defining a chemical structure of a compound including, but not limited to ring structure, chemical bonds, electron pairings, polarities, affinities, charges, hydrophobic vs. hydrophilic properties, etc. For example, a particular type of chemical bond associated with a particular charged atom can be a molecular structural data characteristic that can correspond to a non-exhibited neutral loss (e.g., a neutral loss than can require a higher energy applied to the sample to cause the neutral loss than has been yet applied).

Spectral data can refer to data comprising a plurality of different value types, such as mass per charge ratio (e.g., m/z), conductivity, ion intensity, activation energy, absorbance, etc. For example, a spectrum, resulting from application of activation energy by a spectrometry device, can be graphed as ion intensity or absorbance per m/z.

Neutral loss data can often be at least partially inferred from spectral data. Neutral loss data can refer to a numerical delta value between peaks of spectral data. That is, a neutral loss can refer to an ion, such as water, hydrogen, etc., that is lost from a compound and is not illustrated as a peak m/z value, but rather is instead represented by the spacing, gap, delta, etc. between peaks of spectral data. A neutral loss can be exhibited (and/or expected but not resolved at a particular stage of spectral data corresponding to a particular fragmentation stage or n value of MSⁿ spectra) between adjacent peaks and/or non-adjacent peaks, where such peaks can comprise a precursor and/or fragmentation ions. Note that a fragmentation ion can comprise one or more elements, atom types, etc.

Further, as used herein, chemical data can refer to any one or more of molecular structural data, neutral loss data and/or spectral data. For example, a data aspect is described herein as comprising data describing a known sample in a format that can combine at least neutral loss data and molecular structural data for the known sample.

Accordingly, such correspondences amongst molecular structural data, neutral loss data and/or spectral data can be obtained from known chemical data and a database generated therefrom, from which a model can be trained to recognize such correspondences. A model can be an artificial intelligence (AI) model, such as a machine learning (ML) model. An Al model or ML model employed herein can comprise any one or more types of model including, but not limited to, a neural network, directed neural network, convoluted neural network, image model, language model, etc.

That is, put another way, identification of peaks, neutral losses, etc. can be based on a plurality of considerations including but not limited to any one or more different values and/or reasonings supported by molecular structural data, neutral loss data and/or spectral data employed by such model.

Furthermore, as will be described in detail below, the training of the model, and the execution of the model, can be facilitated by use of a common encoding for the molecular structural data, neutral loss data and/or spectral data. As a result, such various data types can be evaluated by the model, allowing for accurate determinations and/or predictions comprising plural comparative outputs that can be ranked, weighted and/or explained based on the correspondences learned and employed by the model.

As used herein, the phrase "based on" should be understood to mean "based at least in part on," unless otherwise specified.

As used herein, the term "compound" can refer to a single material, multiple materials, composition, sample, solution, product, etc.

As used herein, the term "data" can comprise metadata.

As used herein, the terms "entity," "requesting entity," and "user entity" can refer to a machine, device, component, hardware, software, smart device, party, organization, individual and/or human.

One or more example embodiments are now described with reference to the drawings, where like referenced numerals are used to refer to like drawing elements throughout. In the following description, for purposes of explanation, numerous specific details are set forth in order to provide a more thorough understanding of the one or more example embodiments. It is evident in various cases, however, that the one or more example embodiments can be practiced without these specific details.

Further, it should be appreciated that the embodiments depicted in one or more figures described herein are for illustration only, and as such, the architecture of embodiments is not limited to the systems, devices and/or components depicted therein, nor to any particular order, connection and/or coupling of systems, devices and/or components depicted therein.

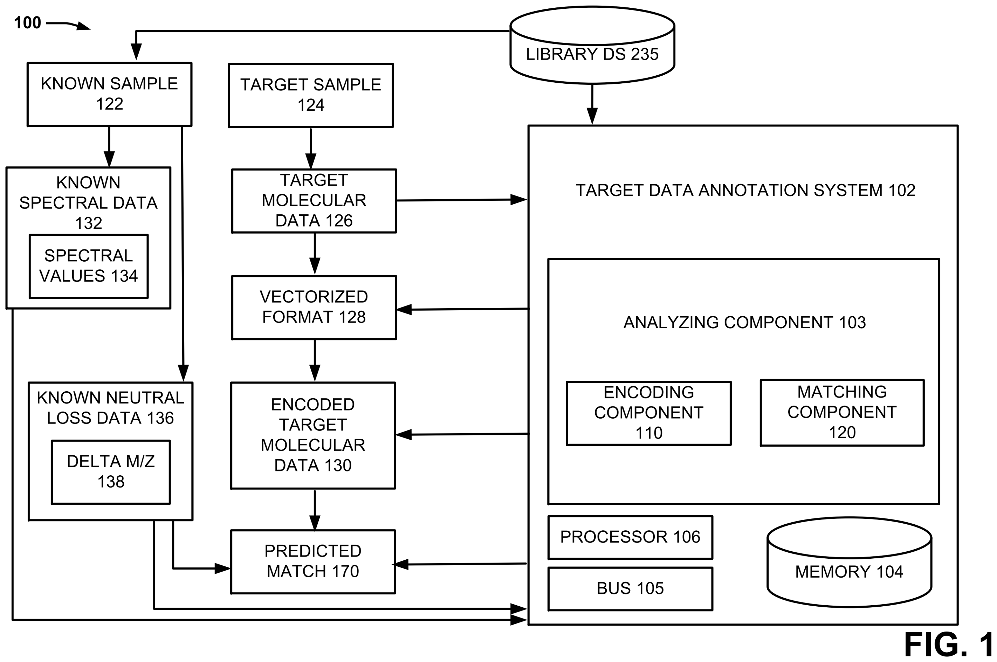

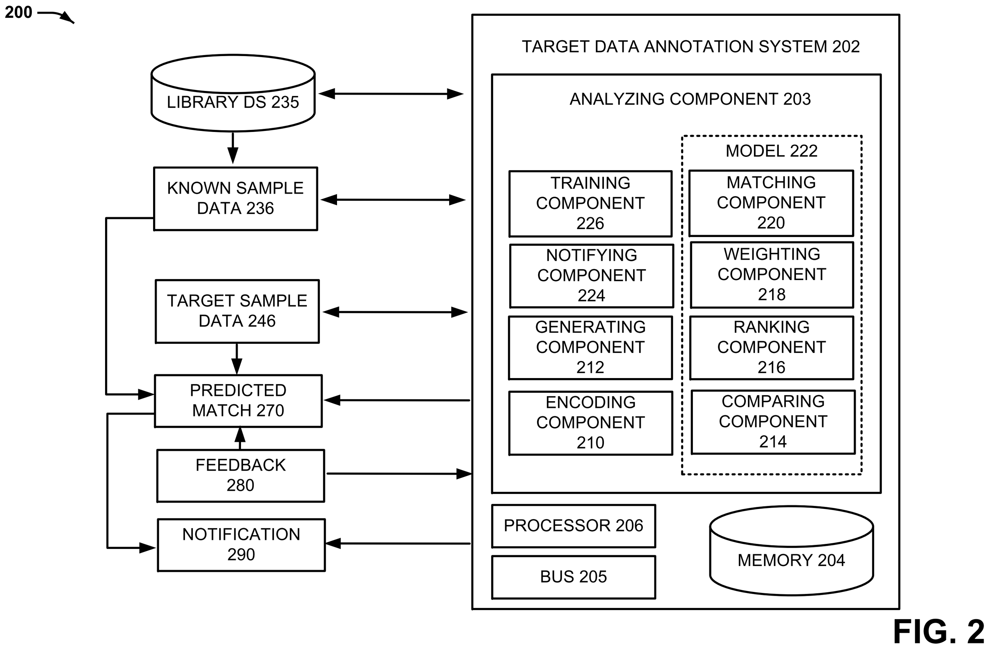

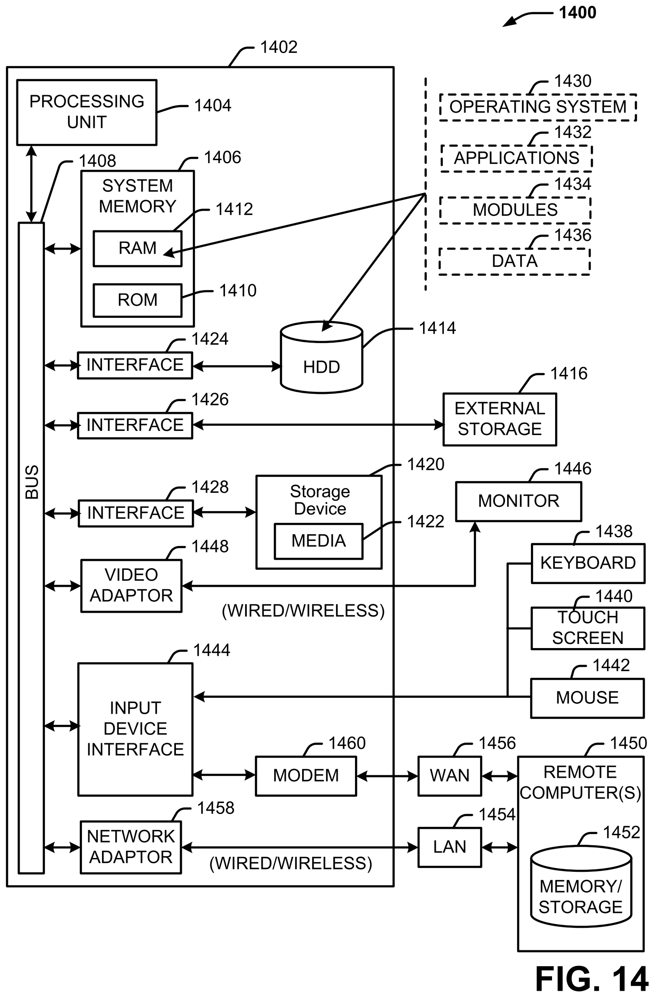

Referring now to FIGS. 1 and 2, in one or more example embodiments, the non-limiting systems 100 and/or 200 illustrated at FIGS. 1 and 2, and/or systems thereof, can further comprise one or more computer and/or computing-based elements described herein with reference to a computing environment, such as the computing environment 1400 illustrated at FIG. 14. In one or more described embodiments, computer and/or computing-based elements can be used in connection with implementing one or more of the systems, devices, components and/or computer-implemented operations shown and/or described in connection with FIGS. 1 and/or 2 and/or with other figures described herein.

Turning first to FIG. 1, the figure illustrates a block diagram of an example, non-limiting system 100 that can comprise a target data annotation system 102 and a library datastore (DS) 135. In one or more embodiments, known chemical data (known spectral data 132, known neutral loss data 136) corresponding to the known sample 122 can be obtained from the library datastore 135. Optionally, the non-limiting system 100 can comprise a measurement device (e.g., a chromatography device, spectrometry device or other scientific measurement device) from which the known chemical data and/or target chemical data (e.g., target molecular data 126) can be obtained. In one or more other embodiments, the measurement device and/or library datastore 135 can be located external to the target data annotation system 102 which can be communicatively coupled to the measurement device and/or library datastore 135.

It is noted that the target data annotation system 102 is only briefly detailed to provide but a lead-in to a more complex and/or more expansive target data annotation system 202 as illustrated at FIG. 2. That is, further detail regarding processes that can be performed by one or more example embodiments described herein will be provided below relative to the non-limiting system 200 of FIG. 2.

Still referring to FIG. 1, the target data annotation system 102 can generally facilitate analysis of target chemical data, resulting in prediction of one or more identifications of fragmentation ions, neutral losses, and/or the target sample itself, based on use of learned correspondences amongst various types of known chemical data. Such known chemical data types can comprise, but are not limited to, known molecular data, known spectral data and/or known neutral loss data.

As used herein, molecular structural data can refer to data defining a chemical structure of a compound including, but not limited to ring structure, chemical bonds, electron pairings, polarities, affinities, charges, hydrophobic vs. hydrophilic properties, etc. For example, a particular type of chemical bond associated with a particular charged atom can be a molecular structural data characteristic that can correspond to a non-exhibited neutral loss (e.g., a neutral loss than can require a higher energy applied to the sample to cause the neutral loss than has been yet applied).

Spectral data can refer to data comprising a plurality of different value types, such as mass per charge ratio (e.g., m/z), conductivity, ion intensity, activation energy, absorbance, etc. For example, a spectrum, resulting from application of activation energy by a spectrometry device, can be graphed as ion intensity or absorbance per m/z.

Neutral loss data can often be at least partially inferred from spectral data. Neutral loss data can refer to a numerical delta value between peaks of spectral data. That is, a neutral loss can refer to an ion, such as water, hydrogen, etc., that is lost from a compound and is not illustrated as a peak m/z value, but rather is instead represented by the spacing, gap, delta, etc. between peaks of spectral data. A neutral loss can be exhibited (and/or expected but not resolved at a particular stage of spectral data corresponding to a particular fragmentation stage or n value of MSⁿ spectra) between adjacent peaks and/or non-adjacent peaks, where such peaks can comprise a precursor and/or fragmentation ions. Note that a fragmentation ion can comprise one or more elements, atom types, etc.

Further, as used herein, chemical data can refer to any one or more of molecular structural data, neutral loss data and/or spectral data. For example, a data aspect is described herein as comprising data describing a known sample in a format that can combine at least neutral loss data and molecular structural data for the known sample.

The target data annotation system 102 can comprise at least a memory 104, bus 105, processor 106, encoding component 110 and/or matching component 120. The processor 106 can be the same as the processor 1404 (FIG. 14), comprised by the processor 1404 or different therefrom. The memory 104 can be the same as the system memory 1406 (FIG. 14), comprised by the system memory 1406 or different therefrom.

Using the above-noted components, the target data annotation system 102 can facilitate a process to execute one or more comparisons of known chemical data to target chemical data, resulting in generation of one or more identifications of one or predicted matches 170. A predicted match 170 can be for a peak of spectral data of the target chemical data, for a neutral loss of neutral loss data of the target chemical data (corresponding to the spectral data of the target chemical data) and/or for the target sample 124 (e.g., precursor) itself. This can be accomplished regardless of whether or not input data has been provided to the system 102 comprising pre-identification of the target sample 124 and/or any of its spectral peaks resulting from analysis of the target sample 124 at a spectrometry device, for example. This also can be accomplished using a vectorized format 128 for encoding the target molecular data 126 corresponding to the target sample 124, which vectorized format 128 can also have been employed for encoding the known chemical data (e.g., known spectral data 132 and/or known neutral loss data 136).

Generally, the encoding component 110 can encode target molecular data 126 for a target sample 124 into a vectorized format 128, resulting in encoded target molecular data 130. For example, one or more atoms, ions, bonds, rings, electron quantities, charges, polarities and/or other structural aspects can be encoded into the vectorized format 128.

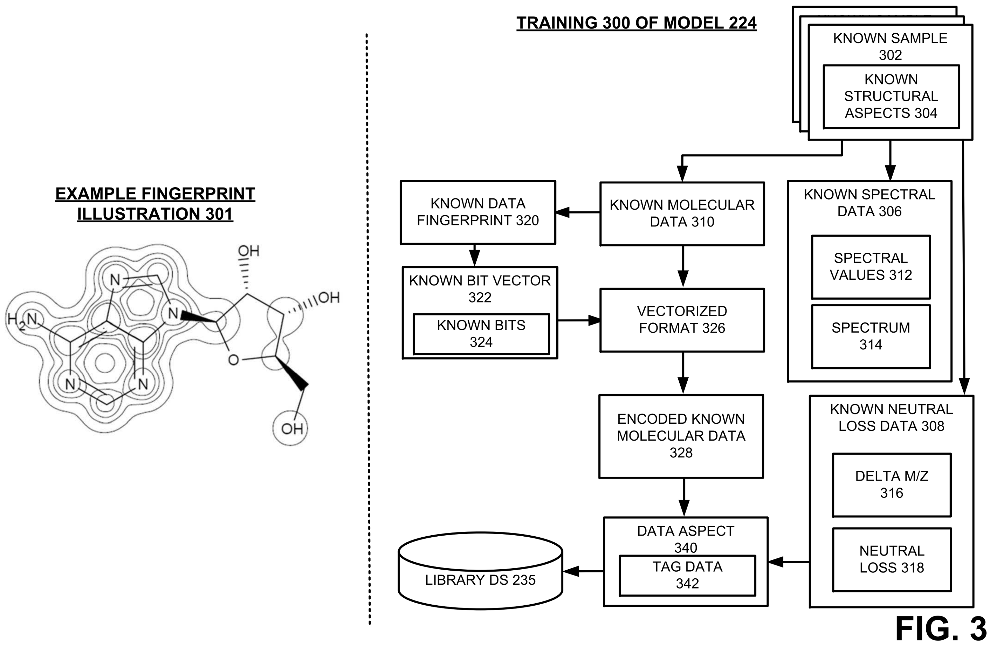

In one or more embodiments, the vectorized format 128 can comprise generation of a bit vector corresponding to the target molecular data 126 by the encoding component 110. The bit vector can be based on a data fingerprint corresponding to the target molecular data 126 and can comprise a plurality of bits representing one or more structural aspects of the target sample 124. An example fingerprint illustration 301 is provided at FIG. 3, illustrating identification of various structural aspects of the corresponding target sample 124 of FIG. 3.

In one or more embodiments, fingerprint data employed can be daylight fingerprint data or other suitable fingerprint data encoded using a Tanimoto index calculation over a bit vector representation of the structural aspects of the target sample 124. That is, the bit vector can be generated encoding one or more structure aspects of the molecules of a target sample 124, and optionally, along with those of other functional group coupled/bonded to the target sample 124.

In one or more cases, the encoding component 110 can be employed to verify that the vectorized format 128 complies with one or more requirements, properties, standards, values, limits, thresholds, etc., such that the encoded target molecular data 130 can be seamlessly compared to known chemical data (e.g., known spectral data 132 and/or known neutral loss data 136).

Using the encoded target molecular data 126, the matching component 120 can generally generate a predicted match 170 of the encoded target molecular data 126 to known neutral loss data 136 for a known sample 122, the known neutral loss data 126 defining a delta mass-to-charge ratio 138 between spectral values 134 of known spectral data 132 corresponding to the known sample 122.

The spectral values 134 can comprise peak values, for example, of adjacent peaks, non-adjacent peaks, and/or peaks corresponding to fragmentation ions and/or precursors.

In one or more embodiments, the known neutral loss data 136 can be comprised by the known spectral data 132. The known neutral loss data 136 and the known spectral data 132 can be provided in any suitable format comprising data and/or metadata. In one or more embodiments, known neutral loss data 136 and/or the known spectral data 132, at least in part, can be comprised in an encoded format, such as the vectorized format 128.

That is, by use of a universal format, such as the encoded or vectorized format, comparison can be made where, previously, in existing frameworks, such search, comparison, identification and/or annotation is not possible. In one non-limiting example, molecular structure data, neutral loss data and/or spectral data, without being limited thereto, can all or partially be encoded in a same vectorized format 128 for a same compound/sample, such as in one or more specified data aspects (e.g., comprising data and/or metadata in any suitable form), thereby allowing for efficient comparison with other such data aspects, and/or with other data (e.g., target compound data) also in the vectorized format 128.

The predicted match 170 can comprise a neutral loss identification. However, in one or more additional and/or alternative cases, the predicted match 170 can additionally and/or alternatively comprise an ion fragment identification, precursor identification and/or target sample identification. Such identification can be based on inferences and/or correspondences amongst different types of data. For example, comparison of known neutral loss data 136 to target neutral loss data for the target sample 124 often does not result in a neutral loss identification as the predicted match 170. Rather, aggregated consideration of peak values, structural values, neutral loss values and/or other data can be aggregated from a known sample 122 being the same or different from the target sample 124, and/or comprising same and/or different fragmentation ions and/or precursors than the target sample 124. Accordingly, a direct comparison, as in existing frameworks, can be inaccurate. Differently, an indirect and inference-based and/or correspondence-based approach employed by the target data annotation system 102, and further described below relative to the target data annotation system 202, can be employed for determining one or more predicted matches 170 having greater accuracy and/or explainability associated therewith.

The encoding component 110 and/or matching component 120 can be operatively coupled to the processor 106 which can be operatively coupled to the memory 104. The bus 105 can provide for the operative coupling. The processor 106 can facilitate execution of the encoding component 110 and/or matching component 120. The encoding component 110 and/or matching component 120 can be stored at the memory 104.

In general, the non-limiting system 100 can employ any suitable method of communication (e.g., electronic, communicative, internet, infrared, fiber, etc.) to provide communication between the target data annotation system 102 and/or any device associated with a user entity, such as the measurement device 150, such as a spectrometry device.

It is noted that one or more measurement devices can be communicatively couplable with the non-limiting system 100 and/or comprised by the non-limiting system 100. For example, a first measurement device can have performed spectrometry analysis on a first compound (target or known compound), and a second measurement device can have performed spectrometry analysis on the first compound or a second compound (another target or known compound). For another example, a first measurement device can have performed spectrometry analysis on a first target compound (e.g., target sample 124) resulting in target molecular data 125 and associated target spectral data, and a second measurement device can have performed spectrometry analysis on the second known compound (e.g., known sample 122) resulting in the known spectral data 132 and associated known molecular data.

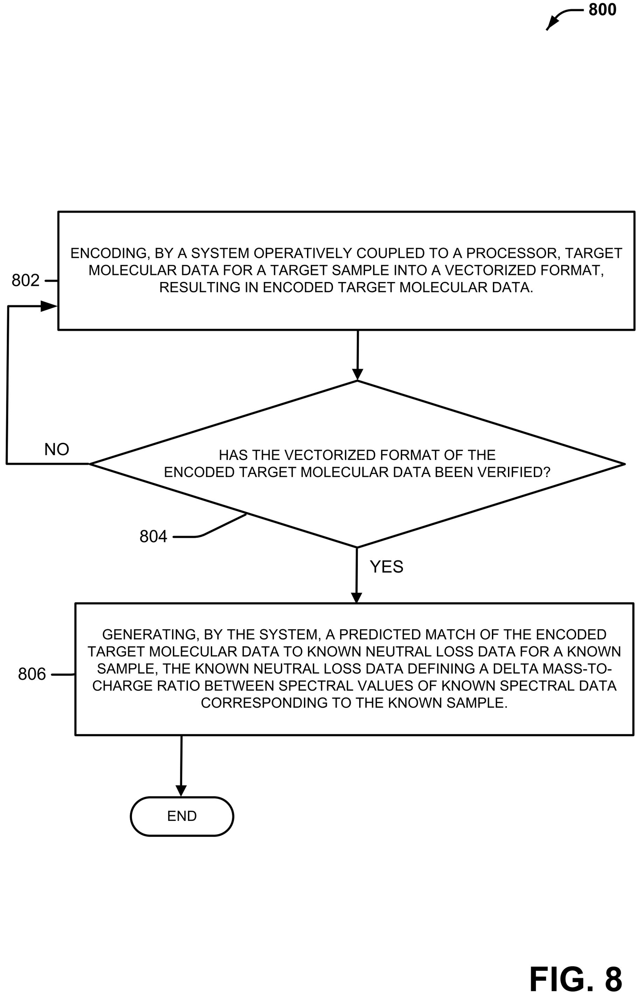

As a summary of the above-described components and functions thereof, referring next only briefly to FIG. 8, illustrated is a flow diagram of an example, non-limiting method 800 that can facilitate a process for chemical data comparison and target data annotation, in accordance with one or more example embodiments described herein, such as the non-limiting system 100 of FIG. 1. While the non-limiting method 800 is described relative to the non-limiting system 100 of FIG. 1, the non-limiting method 800 can be applicable also to other systems described herein, such as the non-limiting system 200 of FIG. 2. Repetitive description of like elements and/or processes employed in respective embodiments is omitted for sake of brevity.

At 802, the non-limiting method 800 can comprise encoding, by a system (e.g., encoding component 110), target molecular data (e.g., target molecular data 126) for a target sample (e.g., target sample 124) into a vectorized format (e.g., vectorized format 128), resulting in encoded target molecular data (e.g., encoded target molecular data 130).

At 804, the non-limiting method 800 can comprise determining, by the system (e.g., encoding component 110 and/or processor 106), whether the vectorized format of the encoded target molecular data has been verified, such as compared to a vectorized format employed for known chemical data to be employed for comparison to the target chemical data. If yes, the non-limiting method 800 can proceed to step 806. If not, the non-limiting method 800 can proceed back to step 802.

At 806, the non-limiting method 800 can comprise generating, by the system (e.g., matching component 120), a predicted match (e.g., predicted match 170) of the encoded target molecular data to known neutral loss data (e.g., known neutral loss data 136) for a known sample (e.g., known sample 122), the known neutral loss data defining a delta mass-to-charge ratio (e.g., delta m/z 138) between spectral values (e.g., spectral values 134) of known spectral data (e.g., known spectral data 132) corresponding to the known sample.

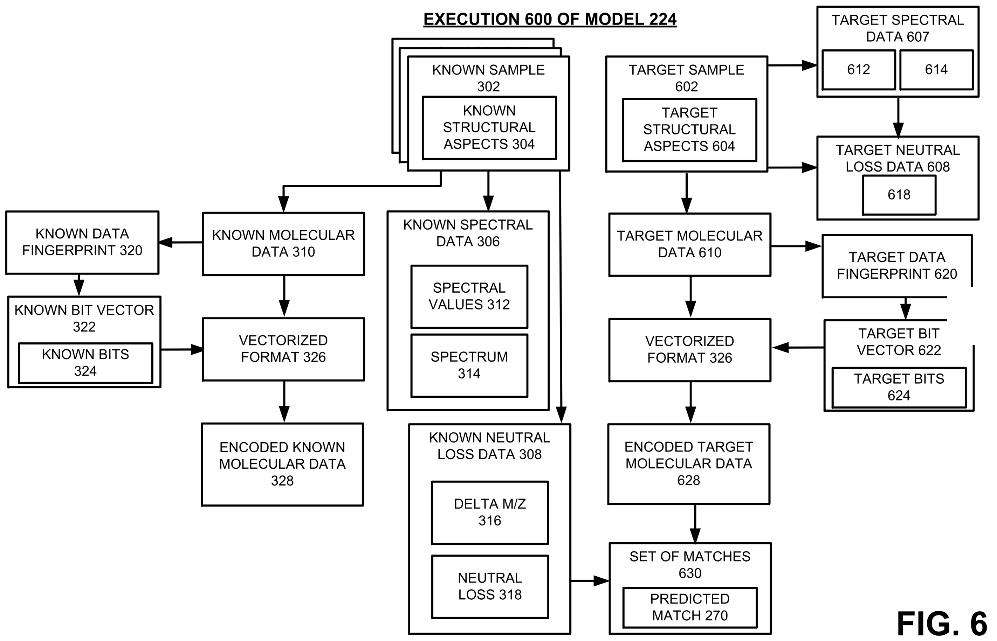

Turning next to FIG. 2, and also referring to FIG. 6, a non-limiting system 200 is illustrated that can comprise a target data annotation system 202 and a library datastore (DS) 235. Repetitive description of like elements and/or processes employed in respective embodiments is omitted for sake of brevity. Description relative to an embodiment of FIG. 1 can be applicable to an embodiment of FIG. 2. Likewise, description relative to an embodiment of FIG. 2 can be applicable to an embodiment of FIG. 1.

In one or more embodiments, the library datastore 235 be separate from but communicatively couplable to the non-limiting system 200.

It is noted that one or more measurement devices can be communicatively couplable with the non-limiting system 200 and/or comprised by the non-limiting system 200. For example, a first measurement device can have performed spectrometry analysis on a first compound (target or known compound), and a second measurement device can have performed spectrometry analysis on the first compound or a second compound (another target or known compound). For another example, a first measurement device can have performed spectrometry analysis on a first target compound (e.g., target sample 602) resulting in target molecular data 610 and associated target spectral data 607, and a second measurement device can have performed spectrometry analysis on the second known compound (e.g., known sample 302) resulting in the known spectral data 306 and associated known molecular data 310.

Generally, the target data annotation system 202 can facilitate analysis of target chemical data (e.g., target sample data 246 comprising target molecular data 610, target spectral data 607 and/or target neutral loss data 608), resulting in prediction (e.g., predicted match 270) of one or more identifications of fragmentation ions, neutral losses, and/or the target sample 602 itself, based on use of learned correspondences amongst various types of known chemical data (e.g., known sample data 236 comprising known spectral data 306, known neutral loss data 308, and/or known molecular data 310). That is, such known chemical data types can comprise, but are not limited to, known molecular data, known spectral data and/or known neutral loss data.

As used herein, molecular structural data can refer to data defining a chemical structure of a compound including, but not limited to ring structure, chemical bonds, electron pairings, polarities, affinities, charges, hydrophobic vs. hydrophilic properties, etc. For example, a particular type of chemical bond associated with a particular charged atom can be a molecular structural data characteristic that can correspond to a non-exhibited neutral loss (e.g., a neutral loss than can require a higher energy applied to the sample to cause the neutral loss than has been yet applied).

Spectral data can refer to data comprising a plurality of different value types, such as mass per charge ratio (e.g., m/z), conductivity, ion intensity, activation energy, absorbance, etc. For example, a spectrum, resulting from application of activation energy by a spectrometry device, can be graphed as ion intensity or absorbance per m/z.

Neutral loss data can often be at least partially inferred from spectral data. Neutral loss data can refer to a numerical delta value between peaks of spectral data. That is, a neutral loss can refer to an ion, such as water, hydrogen, etc., that is lost from a compound and is not illustrated as a peak m/z value, but rather is instead represented by the spacing, gap, delta, etc. between peaks of spectral data. A neutral loss can be exhibited (and/or expected but not resolved at a particular stage of spectral data corresponding to a particular fragmentation stage or n value of MSⁿ spectra) between adjacent peaks and/or non-adjacent peaks, where such peaks can comprise a precursor and/or fragmentation ions. Note that a fragmentation ion can comprise one or more elements, atom types, etc.

Further, as used herein, chemical data can refer to any one or more of molecular structural data, neutral loss data and/or spectral data. For example, a data aspect is described herein as comprising data describing a known sample in a format that can combine at least neutral loss data and molecular structural data for the known sample.

One or more communications between one or more components of the non-limiting system 200 can be provided by wired and/or wireless means including, but not limited to, employing a cellular network, a wide area network (WAN) (e.g., the Internet), and/or a local area network (LAN). Suitable wired or wireless technologies for supporting the communications can include, without being limited to, wireless fidelity (Wi-Fi), global system for mobile communications (GSM), universal mobile telecommunications system (UMTS), worldwide interoperability for microwave access (WiMAX), enhanced general packet radio service (enhanced GPRS), third generation partnership project (3GPP) long term evolution (LTE), third generation partnership project 2 (3GPP2) ultra-mobile broadband (UMB), high speed packet access (HSPA), Zigbee and other 802.XX wireless technologies and/or legacy telecommunication technologies, BLUETOOTH^{®}, Session Initiation Protocol (SIP), ZIGBEE^{®}, RF4CE protocol, WirelessHART protocol, 6LoWPAN (lpv6 over Low power Wireless Area Networks), Z-Wave, an advanced and/or adaptive network technology (ANT), an ultra-wideband (UWB) standard protocol and/or other proprietary and/or non-proprietary communication protocols.

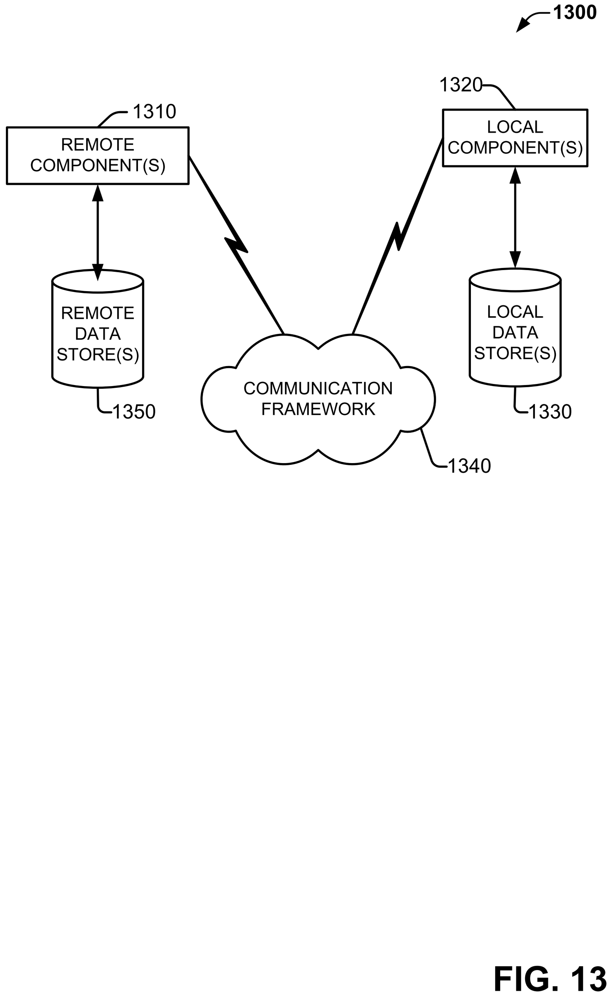

The target data annotation system 202 can be associated with, such as accessible via, a cloud computing environment, such as the cloud computing environment 1300 of FIG. 13.

The target data annotation system 202 can comprise a plurality of components. The components can comprise a memory 204, processor 206, bus 205, encoding component 210, generating component 212, comparing component 214, ranking component 216, weighting component 218, matching component 220, model 222, notifying component 224 and/or training component 226. Using these components, the target data annotation system 202 can facilitate a process to generate one or more predicted matches 270 of one or more fragmentation ions, neutral losses and/or target samples. In one or more embodiments, the target data annotation system 202 can provide one or more such predicted matches 270 in a ranked and/or weighted format. In one or more cases, ranked and/or weighted data can be accompanied by and/or provided separately from one or more correspondence-based (e.g., correspondences among the molecular structure data, neutral loss data and/or spectral data) reasons for the ranked and/or weighted data. This can allow for an understanding of target molecular structure data, neutral loss data and/or spectral data and its causes and/or the reasoning behind any one or more identifications provided by the model.

Discussion next turns to the processor 206, memory 204 and bus 205 of the target data annotation system 202. For example, in one or more example embodiments, the target data annotation system 202 can comprise the processor 206 (e.g., computer processing unit, microprocessor, classical processor, quantum processor and/or like processor). In one or more example embodiments, a component associated with target data annotation system 202, as described herein with or without reference to the one or more figures of the one or more example embodiments, can comprise one or more computer and/or machine readable, writable and/or executable components and/or instructions that can be executed by processor 206 to provide performance of one or more processes defined by such component and/or instruction. In one or more example embodiments, the processor 206 can comprise the encoding component 210, generating component 212, comparing component 214, ranking component 216, weighting component 218, matching component 220, model 222, notifying component 224 and/or training component 226..

In one or more example embodiments, the target data annotation system 202 can comprise the computer-readable memory 204 that can be operably connected to the processor 206. The memory 204 can store computer-executable instructions that, upon execution by the processor 206, can cause the processor 206 and/or one or more other components of the target data annotation system 202 (e.g., encoding component 210, generating component 212, comparing component 214, ranking component 216, weighting component 218, matching component 220, model 222, notifying component 224 and/or training component 226) to perform one or more actions. In one or more example embodiments, the memory 204 can store computer-executable components (e.g., encoding component 210, generating component 212, comparing component 214, ranking component 216, weighting component 218, matching component 220, model 222, notifying component 224 and/or training component 226).

The target data annotation system 202 and/or a component thereof as described herein, can be communicatively, electrically, operatively, optically and/or otherwise coupled to one another via a bus 205. Bus 205 can comprise one or more of a memory bus, memory controller, peripheral bus, external bus, local bus, quantum bus and/or another type of bus that can employ one or more bus architectures. One or more of these examples of bus 205 can be employed.

In one or more example embodiments, the target data annotation system 202 can be coupled (e.g., communicatively, electrically, operatively, optically and/or like function) to one or more external systems (e.g., a non-illustrated electrical output production system, one or more output targets and/or an output target controller), sources and/or devices (e.g., classical and/or quantum computing devices, communication devices and/or like devices), such as via a network. In one or more example embodiments, one or more of the components of the target data annotation system 202 and/or of the non-limiting system 200 can reside in the cloud, and/or can reside locally in a local computing environment (e.g., at a specified location).

In addition to the processor 206 and/or memory 204 described above, the target data annotation system 202 can comprise one or more computer and/or machine readable, writable and/or executable components and/or instructions that, when executed by processor 206, can provide performance of one or more operations defined by such component and/or instruction.

Discussion next turns to the additional components of the target data annotation system 202 (e.g., encoding component 210, generating component 212, comparing component 214, ranking component 216, weighting component 218, matching component 220, model 222, notifying component 224 and/or training component 226). As noted above, generally, the target data annotation system 202 can facilitate a set of processes for identification of one or more neutral losses, fragmentation ions and/or target samples.

These processes can be broken down into a set of processes including, but not limited to training a model 222 using known sample data 236 using the model 222, executing of a comparison of target sample data 246 to the known sample data 236, and generating of a predicted match 270 and corresponding output data also using the model 222.

First, it is noted that in one or more example embodiments, the encoding component 210, generating component 212, comparing component 214, ranking component 216, weighting component 218, matching component 220, model 222, notifying component 224 and/or training component 226 can be implemented independently, without one or more other of the encoding component 210, generating component 212, comparing component 214, ranking component 216, weighting component 218, matching component 220, model 222, notifying component 224 and/or training component 226. Additionally and/or alternatively, the encoding component 210, generating component 212, comparing component 214, ranking component 216, weighting component 218, matching component 220, model 222, notifying component 224 and/or training component 226 can be comprised by a high-level analyzing component 203, one or more of the below-described functions of the encoding component 210, generating component 212, comparing component 214, ranking component 216, weighting component 218, matching component 220, model 222, notifying component 224 and/or training component 226 can be performed by the high-level analyzing component 203, and/or the encoding component 210, generating component 212, comparing component 214, ranking component 216, weighting component 218, matching component 220, model 222, notifying component 224 and/or training component 226 can be omitted with the high-level analyzing component 203 performing one or more of the below-described functions of the one or more omitted encoding component 210, generating component 212, comparing component 214, ranking component 216, weighting component 218, matching component 220, model 222, notifying component 224 and/or training component 226.

As noted above, a first set of one or more processes can comprise training a model 222 using known sample data 236. Accordingly, turning to FIG. 3, and still referring to FIG. 2, one or more data aspects 340 can be generated comprising data/metadata at least partially in a vectorized format 326 for being employed by the training component 226 to train a model 222.

A data aspect 240 can comprise any suitable quantity of data comprising known molecular data 310, encoded known molecular data 328, known spectral data 306 and/or known neutral loss data 308. As used herein, known neutral loss data 308 can be comprised by the known spectral data 306.

The encoding component 210 can obtain known molecular data 310 from a library datastore 235, a standard database, a customer database and/or any suitable output from a spectrometry device. This known molecular data 310 can describe various known structural aspects 304 of a known sample 302. One or more known structural aspects 304 can comprise, but are not limited to, description and/or definition of rings, bonds, electrons, charges, polarities, molecules, atoms, etc.

The encoding component 210 can analyze the raw known molecular data 310 and can encode the known molecular data 310 into the vectorized format 326, resulting in the encoded known molecular data 328.

In one or more embodiments, the vectorized format 326 can comprise generation of a bit vector 322 from known fingerprint data 320 corresponding to the known molecular data 310 by the encoding component 210. The bit vector 322 can be based on the data fingerprint 320 and can comprise a plurality of bits 324 representing one or more structural aspects 304 of the known sample 302. An example fingerprint illustration 301 is provided at FIG. 3, illustrating identification of various structural aspects of a corresponding sample.

In one or more embodiments, fingerprint data 320 employed can be daylight fingerprint data or other suitable fingerprint data encoded using a Tanimoto index calculation over a bit vector representation of the structural aspects 304 of the known sample 302. That is, the bit vector 322 can be generated encoding one or more structural aspects 304 of the molecules of a known sample 302, and optionally, along with those of other functional groups coupled/bonded to the known sample 302.

In one or more cases, the encoding component 210 can be employed to verify that the vectorized format 326 complies with one or more requirements, properties, standards, values, limits, thresholds, etc., such that the encoded known molecular data 328 can be seamlessly integrated with and/or compared to other known chemical data (e.g., for one or more other known samples 302).

In one or more cases, the encoding component 210 further can obtain known spectral data 306 and/or known neutral loss data 308. The known spectral data 306 can comprise peak values (e.g., m/z values) for fragmentation ions and/or precursors per intensity value. Gaps between peaks, whether or not adjacent to one another, can, but not always, represent neutral losses of molecules and/or atoms lost from the known sample 302 during application of fragmenting energy to the known sample 302 by a spectrometry device. For example, a neutral loss of 18 m/z can, in one or more cases, represent H₂O or water loss from the known sample 302.

It is noted that this particular neutral loss, in a particular location of the known spectral data 306, representing a particular order of fragmentation of the known sample 302, can be employed to define one or more inferences, such as other neutral loss identifications and/or precursor/fragmentation ion identifications of the known spectral data 306.

In one or more cases, such neutral loss data 308 comprising one or more delta m/z values 316 corresponding to one or more neutral losses 318 can be obtained by the encoding component 210.

In one or more other cases, such neutral loss data 308 comprising one or more delta m/z values 316 corresponding to one or more neutral losses 318 can be generated by the generating component 212, based on the known spectral data 306. That is, a neutral loss 318 can be defined by a delta m/z value 316 between a pair of spectral peak values 312, such as illustrated at a spectrum 314 of the known spectral data 306. The pair of spectral peak values 312 can correspond to fragmentation ions and/or precursors that are adjacent to one another and/or non-adjacent to one another.

In one or more cases, the generating component 212 can generate neutral loss data 308 that is non-exhibited at a spectrum 314 defined by the known spectral data 306. That is, such non-exhibited neutral losses 318 can fail to appear during fragmentation such as due to a chemical structure (e.g., chemical bond type), chemical property, fragmentation energy not being reached, etc. Put another way, the non-exhibited neutral loss can correspond to an ion that has not fragmented from a target sample due to a same and/or different reason (e.g., chemical structure, chemical property, fragmentation energy). At this stage of the training, this data is not inferred, but rather can be directly obtained such as being part of the known neutral loss data 308 and/or known spectral data 306 and/or being provided as input by a user entity employing a computing device communicatively couplable to the target data annotation system 202.

In one or more cases, the known neutral loss data 308 can be non-encoded and thus not in the vectorized format 326.

Based on the input and/or generation of the known neutral loss data 308 and encoding of the encoded known molecular data 328 all for a known sample 302, the generating component 212 can generate tag data 342 linking the known neutral loss data 308 to the encoded known molecular data 328. That is, neutral losses 318 can be matched to structural aspects 204, using tags, links, labels, tables, matrices, nodes and edges, and/or any other tag data 342. In this way, the known neutral loss data 308 can be at least partially provided in the vectorized format 326, whether directly and/or via reference through the use of the tag data 342. In one or more cases, the encoding component 210 can aid the generating component 212 by encoding one or more aspects of the neutral loss data 308 into a bit vector 322 and thus into the vectorized format 326.

Finally, the generating component 212 can generate one or more data aspects 340 aggregating the encoded known molecular data 328, known neutral loss data 308 (whether or not in the vectorized format 326) and tag data 342 corresponding to the known sample. Such data aspect 340 can be stored, by the generating component 212 at the library datastore 235 or at any other suitable location communicatively couplable and/or accessible to the target data annotation system 202.

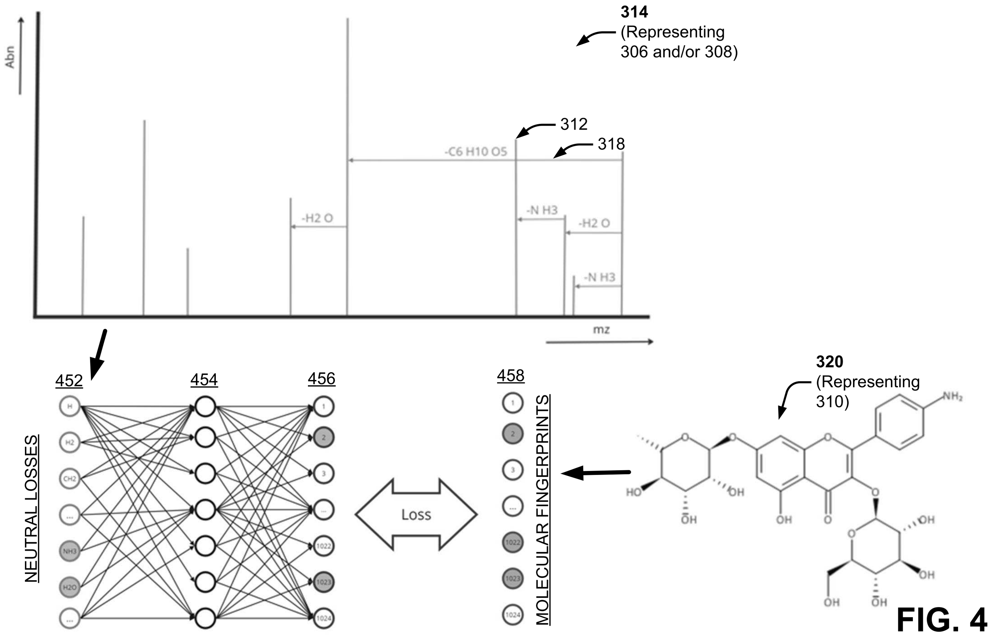

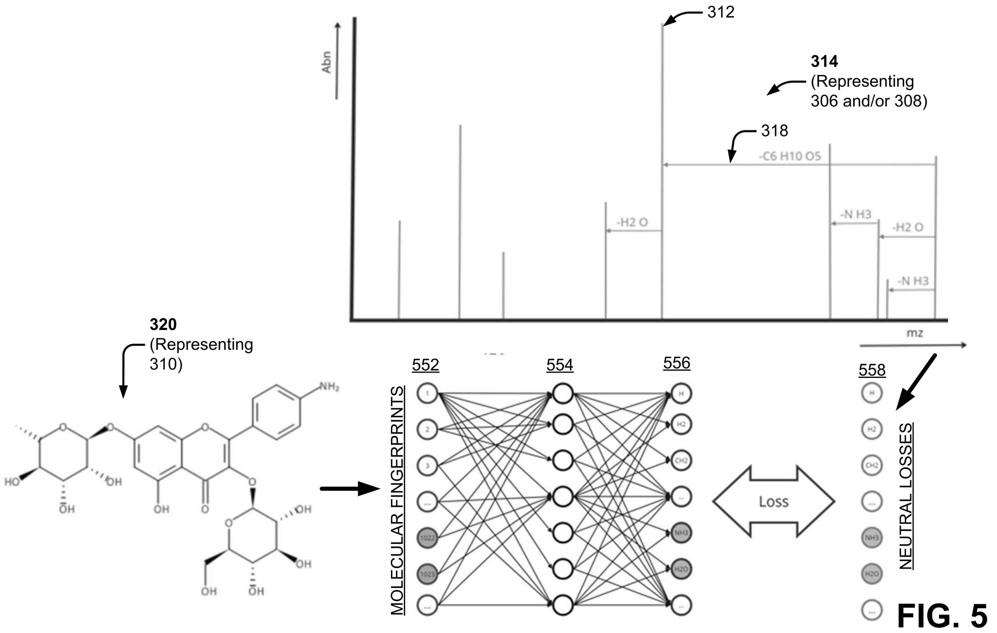

Discussion next turns to the training component 226 and to FIGS. 4 and 5. Briefly, the training component 226 can employ a plurality of data aspects 340 for different known samples 302, such as broken into known, verification and/or training groups of data aspects 340, to train a model 222. In connection therewith, one or more additional aspects of data, such as tag data and/or additional neutral loss data 308 corresponding to one or more non-exhibited neutral losses 318 can be generated, as discussed below.

A model 222 can be an artificial intelligence (AI) model, such as a machine learning (ML) model. An Al model or ML model 322 employed herein can comprise any one or more types of model including, but not limited to, a neural network, directed neural network, convoluted neural network, image model, language model, etc.

Accordingly, generally, the training component 226 can train one or more models 222 with a set of data aspects 304 comprising encoded known molecular data 328, corresponding known neutral loss data 308 and corresponding tag data 342.

For example, looking first to FIG. 4, a model 222 can be trained to obtain input neutral loss data and to match such input neutral loss data to encoded molecular structural data. At FIG. 4, illustrated is a spectrum 314 comprising spectral values 312 and which is based on known spectral data 306 for a known sample 302. As illustrated at the known spectral data 306 is known neutral loss data 308 illustrated as one or more neutral losses 318. Such neutral losses 318 can be exhibited and can be detected by the generating component 212, for example. Also illustrated at FIG. 4 is known molecular data 310 represented by a known data fingerprint 320 of the known sample 302.

The model 222 can be directed, such as by the training component 226, based on the data input to the model 222 by the training component 226, to generate a set of input neurons 452 and hidden neurons 454 corresponding to known neutral loss data 308 and additional input neurons 458 corresponding to encoded known molecular structural data 310. The model 222 can, in response, generate a set of one or more output neurons 456 comprising aggregated encoded known molecular structural data 310 and neutral loss data 308. The output neurons 456 can be tagged to and/or comprised by the one or more data aspects 340 for the known sample 302.

Turning next to FIG. 5, the model 222 also can be trained to obtain input encoded molecular structural data and to match such input encoded molecular structural data to neutral loss data. At FIG. 5, illustrated is a known data fingerprint 320 of the known sample 320 representing the known molecular data 310. Also illustrated at FIG. 5 is known spectral data 306 and/or known neutral loss data 308 illustrated as one or more neutral losses 318 at a spectrum 314.

The model 222 can be directed, such as by the training component 226, based on the data input to the model 222 by the training component 226, to generate a set of input neurons 552 and hidden neurons 554 corresponding to encoded known molecular structural data 310 and additional input neurons 558 corresponding to known neutral loss data 308. The model 222 can, in response, generate a set of one or more output neurons 556 comprising aggregated encoded known molecular structural data 310 and neutral loss data 308. The output neurons 556 can be tagged to and/or comprised by the one or more data aspects 340 for the known sample 302.

It is noted that while FIGS. 4 and 5 are related to a model 222 comprising and/or being a neural network, one or more other types of models, such as other correlation models, can be employed and/or comprised by the model 222. As such, the illustrations and explanation directed to FIGS. 4 and 5 are non-limiting and are meant to illustrate, more generally, the aggregation of different types of data by the model 222 during training of the model 222 as facilitated by the training component 226.

It also is noted, that as mentioned above, and as illustrated at the neuron illustrations at FIGS. 4 and 5, in one or more cases, one or more additional learned neutral losses 318, such as non-exhibited neutral losses 318, can be learned by the model 222 in view of the generation of the one or more data aspects 340. That is, in view of the aggregated data linking molecular structural features and neutral losses in at least a partially vectorized format 326, the one or more additional neutral losses 318 can be learned, such as by data overlap, inherency, matched correspondences, etc.

Additionally, and/or alternatively, in one or more embodiments, the training component 226 can facilitate a feedback evaluation relative to the one or more output predicted matches 270. For example, this can comprise input of data requesting or changing of one or more weights for one or more model hyperparameters for one or more trained models 222 by a user entity (e.g., using a computing device that is communicatively couplable to the non-limiting system 200).

Discussion next turns to a second set of processes for executing the trained model 222 resulting in prediction of one or more predicted matches 270.

That is, looking to FIG. 6, and also still to FIG. 2, it can be desired to employ the trained model 222 to generate one or more predicted matches 270 relative to one or more target samples 602. For example, a desired identification can comprise a target sample identification, precursor identification, fragmentation peak identification and/or neutral loss identification, any of which can be comprised by one or more predicted matches 270 to be generated by the trained model 222 in connection with the target data annotation system 202.

The encoding component 210 can obtain target molecular data 610 from a library datastore 235, a standard database, a customer database, any suitable output from a spectrometry device and/or any other computer device associated with a user entity and communicatively couplable to the non-limiting system 200. This target molecular data 610 can describe various target structural aspects 604 of a target sample 602. One or more target structural aspects 604 can comprise, but are not limited to, description and/or definition of rings, bonds, electrons, charges, polarities, molecules, atoms, etc.

The encoding component 210 can analyze the raw target molecular data 610 and can encode the target molecular data 610 into the vectorized format 326, resulting in the encoded target molecular data 628.

In one or more embodiments, the vectorized format 326 (e.g., the same described above relative to use by the non-limiting system 200 with the known sample data 236, can comprise generation of a bit vector 622 from target fingerprint data 620 corresponding to the target molecular data 610 by the encoding component 210. The bit vector 622 can be based on the data fingerprint 620 and can comprise a plurality of bits 624 representing one or more structural aspects 604 of the target sample 602. An example fingerprint illustration 301 is provided at FIG. 3, illustrating identification of various structural aspects of a corresponding sample.

In one or more embodiments, fingerprint data 620 employed can be daylight fingerprint data or other suitable fingerprint data encoded using a Tanimoto index calculation over a bit vector representation of the structural aspects 604 of the target sample 602. That is, the bit vector 622 can be generated encoding one or more structural aspects 604 of the molecules of a target sample 602, and optionally, along with those of other functional groups coupled/bonded to the target sample 602.

In one or more cases, the encoding component 210 can be employed to verify that the vectorized format 326 complies with one or more requirements, properties, standards, values, limits, thresholds, etc., such that the encoded target molecular data 628 can be seamlessly integrated with and/or compared to other target chemical data (e.g., for one or more other target samples 602 and/or known sample 302).

In this way, the one or more example embodiments described herein can be employed to employ encoding of data in a universal format employable for search, comparison, identification and/or annotation of molecular data, spectral data and/or neutral loss data relating to a plurality of compounds. That is, by use of a universal format, such as an encoded or vectorized format, to be discussed below, comparison can be made where, previously, in existing frameworks, such search, comparison, identification and/or annotation is not possible. In one non-limiting example, molecular structure data, neutral loss data and/or spectral data, without being limited thereto, can all or partially be encoded in a same vectorized format for a same compound, such as in one or more specified data aspects (e.g., comprising data and/or metadata in any suitable form), thereby allowing for efficient comparison with other such data aspects, and/or with other data (e.g., target compound data) also in the vectorized format.

In one or more cases, the encoding component 210 further can obtain target spectral data 606 and/or target neutral loss data 608. The target spectral data 606 can comprise peak values (e.g., m/z values) for fragmentation ions and/or precursors per intensity value. Gaps between peaks, whether or not adjacent to one another, can, but not always, represent neutral losses of molecules and/or atoms lost from the target sample 602 during application of fragmenting energy to the target sample 602 by a spectrometry device. For example, a neutral loss of 18 m/z can, in one or more cases, represent H₂O or water loss from the target sample 602.

It is noted that this particular neutral loss, in a particular location of the target spectral data 606, representing a particular order of fragmentation of the target sample 602, can be employed to define one or more inferences, such as other neutral loss identifications and/or precursor/fragmentation ion identifications of the target spectral data 606.

In one or more cases, such neutral loss data 608 comprising one or more delta m/z values 616 corresponding to one or more neutral losses 618 can be obtained by the encoding component 210.

In one or more other cases, such neutral loss data 608 comprising one or more delta m/z values 616 corresponding to one or more neutral losses 618 can be generated by the generating component 212, based on the target spectral data 606. That is, a neutral loss 618 can be defined by a delta m/z value 616 between a pair of spectral peak values 612, such as illustrated at a spectrum 614 of the target spectral data 606. The pair of spectral peak values 612 can correspond to fragmentation ions and/or precursors that are adjacent to one another and/or non-adjacent to one another.

In one or more cases, the generating component 212 can generate target neutral loss data 608 that is non-exhibited at a spectrum 614 defined by the target spectral data 606. That is, such non-exhibited neutral losses 618 can fail to appear during fragmentation such as due to a chemical structure (e.g., chemical bond type), chemical property, fragmentation energy not being reached, etc. Put another way, the non-exhibited neutral loss can correspond to an ion that has not fragmented from a target sample due to a same and/or different reason (e.g., chemical structure, chemical property, fragmentation energy). At this stage of the training, this data is not inferred, but rather can be directly obtained such as being part of the target neutral loss data 608 and/or target spectral data 606 and/or being provided as input by a user entity employing a computing device communicatively couplable to the target data annotation system 202.

Using the target sample data 246 (e.g., target molecular data 610, encoded target molecular data 628, target neutral loss data 608 and/or target spectral data 607) as a first input, and using the aforediscussed known sample data 236, the comparing component 214, ranking component 216, weighting component 218 and/or matching component 220 can perform one or more processes.

In one or more cases, one or more of the comparing component 214, ranking component 216, weighting component 218 and/or matching component 220 can be comprised by the model 222. In one or more other cases, one or more processes described below as being performed by the one or more of the comparing component 214, ranking component 216, weighting component 218 and/or matching component 220 can be performed by the model 222. In one or more cases, one or more of the comparing component 214, ranking component 216, weighting component 218 and/or matching component 220 can be omitted with the processes performed thereby aggregated into functionality of the model 222. In one or more cases, one or more of the comparing component 214, ranking component 216, weighting component 218 and/or matching component 220 can be non-physical components representing one or more functionalities of the model 222.

Turning first to the comparing component 214, this component can generally compare known sample data 236 to target sample data 246. In one or more embodiments, the comparing component 214 can compare like types of data (e.g., encoded known molecular data 328 to encoded target molecular data 628) and/or non-like types of data, e.g., comparing known neutral loss data 308 to the encoded target molecular data 628). Regarding this latter example, a neutral loss 318 of the known neutral loss data 308 can be matched to and/or compared to a structural feature (e.g., target structural aspect 604) of the encoded target molecular data 628 corresponding to an ion lost via the neutral loss 318.

For example, the one or more example embodiments described herein can be employed to leverage information related to one or more compounds different from a target compound for which annotation is desired. That is, a prediction regarding identification of a neutral loss (exhibited or not exhibited in spectral data), a prediction regarding identification of an ion based on a chemical structure (e.g., chemical bond type), and/or a prediction regarding identification of a target compound, without being limited thereto, can be made based on molecular structure data, neutral loss data and/or spectral data, without being limited thereto, that corresponds to a known compound different from the target compound. Such known compound can be of a same family, chemical category type, etc., and/or can have one or more structural features, ions and/or neutral losses in common with the target compound, for example.

In one or more cases, comparison can certainly comprise aggregated known sample data 236 (e.g., as comprised by a data aspect 340) against any one or more types of target sample data 246. That is, this can be a more potent benefit provided by the one or more embodiments described herein as compared to existing frameworks.

For example, the one or more example embodiments described herein can be employed to generate inferences and/or neutral loss data 608 corresponding to a target sample 602, via the aforementioned comparison, that would not otherwise be available by merely comparing molecular data and/or neutral loss data for the target sample 602 to a library of known molecular data 310 (e.g., non-encoded) and/or known neutral loss data 308 (e.g., non-encoded).

As another example, based on spectral data (regardless of whether target and/or known) defining a spectrum, one or more neutral losses can be exhibited, while one or more other neutral losses can be non-exhibited. That is, such non-exhibited neutral losses can fail to appear during fragmentation such as due to a chemical structure (e.g., chemical bond type), chemical property, fragmentation energy not being reached, etc. Put another way, the non-exhibited neutral loss can correspond to an ion that has not fragmented from a target sample due to a same and/or different reason (e.g., chemical structure, chemical property, fragmentation energy). Such non-exhibited neutral loss can be determined as corresponding to a target sample 602 by employing aggregated known sample data 236, such as of one or more data aspects 340 corresponding to one or more known sample 302 that can be the same as and/or different from the target sample 602.

For example, while a known sample 302 different from the target sample 602 can comprise different ions, structure aspects and/or molecules, inferences can be made based on similarities, such as due to one or more structure aspects 304, 604 (e.g., bond type and/or location, etc.) resulting in an inference of a non-exhibited neutral loss that is predicted to correspond to the target sample 602.

Based on the comparison provided by the comparing component 214, the matching component 220 can generally generate a predicted match 270 of at least one aspect of the known sample data 236 to the target sample data 246. For example, the matching component 220 can generate a predicted match 270 based on the encoded target molecular data 628 to known neutral loss data 308 for the known sample 302, the known neutral loss data 308 defining a delta mass-to-charge ratio 316 between spectral values 312 of known spectral data 306 corresponding to the known sample 302. That is, in correspondence with discussion directly above, this predicted match 270 can be based on use of encoded aggregated data for the known sample 302 (e.g., of one or more data aspects 340).

As another example, the matching component 220 can match the known neutral loss data 308 to a known bit 624 of a target bit vector 622.

As also noted above, a predicted match 270 can comprise one or more identifications, such as an identification of a neutral loss corresponding to the target sample 602, such as a non-exhibited neutral loss (e.g., a neutral loss not exhibited at a known spectrum 614). Additionally, and/or alternatively, a predicted match 270 can comprise an identification of a target sample 602, precursor corresponding to the target sample 602 and/or fragmentation ion corresponding to the target sample 602.

In one or more particular cases, a predicted match 270 can comprise a neutral loss identification. However, in one or more additional and/or alternative cases, the predicted match 270 can additionally and/or alternatively comprise an ion fragment identification, precursor identification and/or target sample identification.

Again, any one or more such identifications can be based on inferences and/or correspondences amongst different types of known sample data 236. For example, comparison of known neutral loss data 308 to target neutral loss data for the target sample 602 often does not result in a neutral loss identification as the predicted match 270. Rather, aggregated consideration of peak values, structural values, neutral loss values and/or other data can be aggregated from a known sample 302 being the same or different from the target sample 602, and/or comprising same and/or different fragmentation ions and/or precursors than the target sample 602. Accordingly, a direct comparison, as in existing frameworks, can be inaccurate. Differently, an indirect and inference-based and/or correspondence-based approach employed by the target data annotation system 202, can be employed for determining one or more predicted matches 270 having greater accuracy and/or explainability associated therewith.

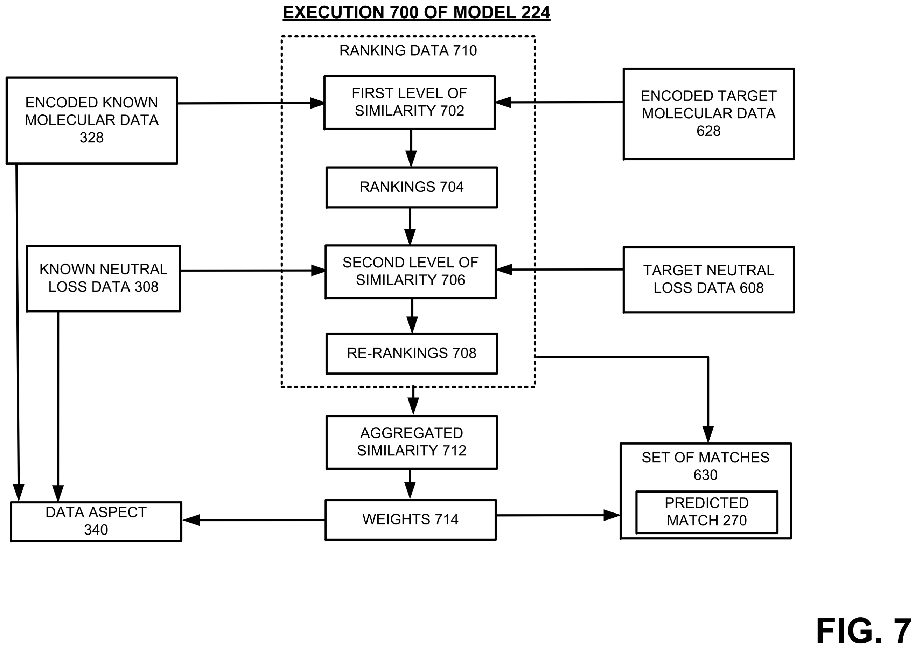

Turning now to FIG. 7, and still referring to FIGS. 2 and 6, in connection with the comparing component 214, one or more predicted matches 270 can additionally and/or alternatively be generated using a system of rankings 704, re-rankings 708 and/or weights 714.

As used herein, a ranking 704 and/or re-ranking 708 can refer to an annotated ordering of identifications based on a level of similarity of a target fragmentation ion, target neutral loss and/or target sample to a corresponding known fragmentation ion, known neutral loss and/or known sample. It is noted that ranking need not be for same type to same type, e.g., sample to sample. Rather, a ranking of similarity of a neutral loss to a sample, for example, can represent an inference of similarity therebetween.

Differently, a weight 714 can refer to a quantitative annotation of predicted accuracy of a predicted match 270, based on the aggregated known sample data 236 employed to generate the match 270, such as by the matching component 220 and/or trained model 222.

Accordingly, for an example, the ranking component 216 can generate rankings 704 for a set of one or more known samples 302 based on a first level of similarity 702 of encoded known molecular data 328, corresponding to the one or more known samples 302, to the encoded target molecular data 628. Accordingly, the rankings 704 can define an order of similarity (e.g., with a highest ranking referring to highest similarity) based on compared encoded molecular data 328, 628. Rankings 704 can refer to similarity of particular identifications and/or to similarity of known samples 302 to target sample 602 based on the various identifications.

Where such ranking is employed, the comparing component 214 can compare the known neutral loss data 308 and target neutral loss data 608, for the target sample 602, resulting in a set of one or more possible matches (e.g., of a set of matches 630), from which the matching component 220 can generate a predicted match 270, of one or more known samples 302 (e.g., of data thereof) corresponding to the target sample 602.

In connection therewith, the ranking component 216 can further generate one or more re-rankings 708 of the set of one or more known samples 302 (e.g., of data thereof) based on a second level of similarity 706 of known neutral loss data 308 corresponding to the set of one or more known samples 302, to target neutral loss data 608 corresponding to the target sample 602. For example, the second level of similarity 706 can be applied to neutral loss data, of the known neutral loss data 308, that corresponds to known bits 324, of the encoded known molecular data 328, that match to target bits 624 of the encoded target molecular data 628. Accordingly, the re-rankings 708 can define an order of similarity (e.g., with a highest ranking referring to highest similarity) using the particular set of matches 630 resulting from the initial rankings 704 as a base and/or starting point. Re-rankings 708 can refer to similarity of particular identifications and/or to similarity of known samples 302 to target sample 602 based on the various identifications.

It is noted that, in one or more embodiments, the re-ranking can be performed without the ranking. As such, no ranking data (employing rankings 704) would be available as a starting point for the re-ranking.

In connection with the comparing component 214 and/or ranking component 216, the weighting component 218 can generate a weight 714 for a data aspect 340 corresponding to the known sample 302, where the weight 714 is generated based on an aggregated similarity between the encoded known molecular data 328 and the encoded target molecular data 628, and between target neutral loss data 608 and the known neutral loss data 308. Such weights 714 can be based solely on a comparison of aggregated data, and/or can take the rankings 704 and/or re-rankings 708 into consideration as part of an employed calculation and/or algorithm.

That is, weighting can result from the ranking and re-ranking, just from the re-ranking, and/or can be in place of the ranking/re-ranking.

An example weight 714 can range between a 0 and a 1, with 1 representing high accuracy and 0 representing little to no accuracy, although other suitable ranges can be employed.

Comparing, ranking, re-ranking and/or weighting can be performed in any suitable order and/or at least partially at a same time as one another.

Following therefrom, based on one or more outputs of the comparing component 214, ranking component 216, and/or weighting component 218, the matching component 220 can generate one or more predicted matches 270. This generation can be based on output of the comparing component 214 alone and/or can employ one or more rankings, 704, re-rankings 708 and/or weights 714.

For example, in one or more embodiments, one or more predicted matches 270 can be obtained for different and/or same identifications. For example, a set of matches 630 can be output by the matching component 220, as explained above. That is, in one or more embodiments, a group of two or more predicted matches 270 corresponding to a same identification, such as an identification of a neutral loss, can comprise one or more of the re-rankings 704. In one or more embodiments, a group of two or more predicted matches 270 corresponding to a same identification, such as an identification of a neutral loss, can comprise one or more of the weights 714. In one or more other embodiments, two or more identifications can be mutually exclusive (e.g., can contradict one another).

Discussion next refers still to FIG. 7 and FIG. 2, and to a third set of processes for further executing the trained model 222 to output one or more additional outputs accompanying the one or more predicted matches 270.

Accompanying the one or more predicted matches 270 can be one or more notifications 290 output by the notifying component 224. Generally, the notifying component 224 can generate report data comprising cause data linking an identification to one or more aspect of known sample data 236. For example, in one or more cases, the notifying component 224 can generate report data comprising cause data linking a structural feature (e.g., structural aspect 604) of the target sample 602 to specified neutral loss data 318, of the known neutral loss data 308, corresponding to at least one or more bits 324 of the encoded known molecular data 328. This can allow for an understanding of target molecular structure data, neutral loss data and/or spectral data and its causes and/or the reasoning behind any one or more identifications provided by the model.

For another example, in one or more cases, a notification 290 can comprise the ranked, weighted and/or non-ranked/non-weighted data accompanied by and/or provided separately from one or more correspondence-based (e.g., correspondences among the molecular structure data, neutral loss data and/or spectral data) reasons for the ranked and/or weighted data. This also can allow for an understanding of target molecular structure data, neutral loss data and/or spectral data and its causes and/or the reasoning behind any one or more identifications provided by the model.

In one or more embodiments, the notifying component 224 can generate a visual (e.g., can generated display data that can be displayed at a graphical user interface communicatively couplable to the non-limiting system 200) of a spectrum, molecule and/or fingerprint having one or more aspects thereof being labeled and/or tagged with an identification and/or explainability (e.g., cause of identification).

In one or more embodiments, the model 222, notifying component 224 and/or training component 226 can facilitate generation of and/or modification of a data aspect 340, stored at and/or to be stored at the library datastore 250 and/or other suitable location, such as to be employed by the model 222 and/or target data annotation system 202 for future identifications, trainings, etc. In one or more embodiments, such generation and/or modification can comprise generation of tag data linking the known neutral loss data 308 to the encoded target molecular data 628, such as at a data aspect for the known sample 302 and/or for the target sample 602.

In one or more embodiments, the training component 226 can facilitate a feedback evaluation relative to the one or more output predicted matches 270. For example, this can comprise input of data requesting or changing of one or more weights for one or more model hyperparameters for one or more trained models 222 by a user entity (e.g., using a computing device that is communicatively couplable to the non-limiting system 200).

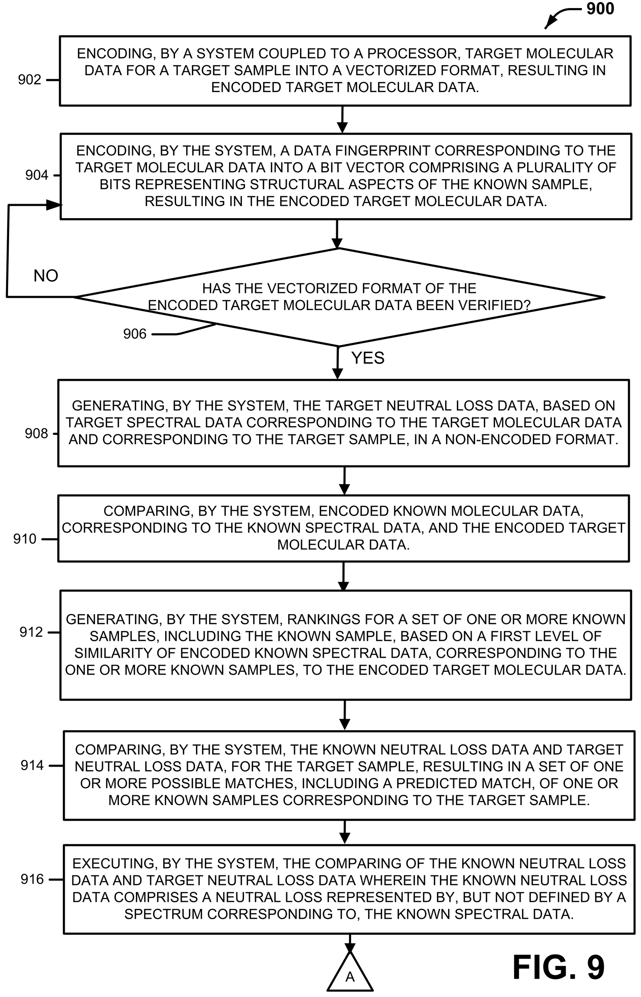

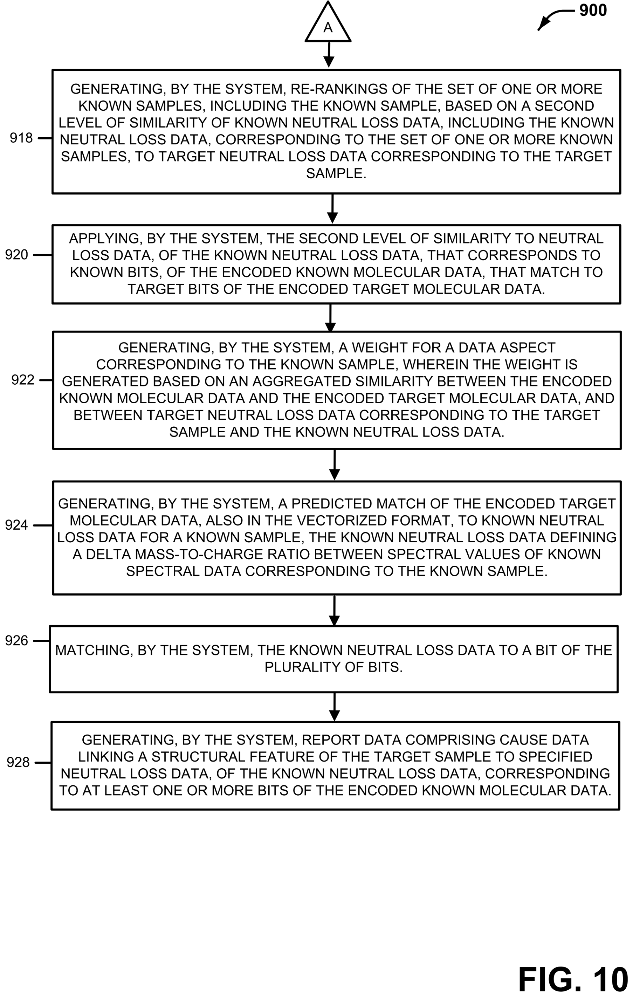

As a summary of the above-described components and/or functions thereof, referring next to FIGS. 9 and 10, illustrated is a flow diagram of an example, non-limiting method 900 that can facilitate a process for chemical data comparison and target data annotation, in accordance with one or more example embodiments described herein, such as the non-limiting system 200 of FIG. 2. While the non-limiting method 900 is described relative to the non-limiting system 200 of FIG. 2, the non-limiting method 900 can be applicable also to other systems described herein, such as the non-limiting system 100 of FIG. 1. Repetitive description of like elements and/or processes employed in respective embodiments is omitted for sake of brevity.

At 902, the non-limiting method 900 can comprise encoding, by a system (e.g., encoding component 210) target molecular data for a target sample into a vectorized format, resulting in encoded target molecular data.

At 904, the non-limiting method 900 can comprise encoding, by the system (e.g., encoding component 210), a data fingerprint corresponding to the target molecular data into a bit vector comprising a plurality of bits representing structural aspects of the known sample, resulting in the encoded target molecular data.

At 906, the non-limiting method 900 can comprise determining, by the system (e.g., encoding component 210 and/or processor 206), whether the vectorized format of the encoded target molecular data has been verified, such as compared to a vectorized format employed for known chemical data to be employed for comparison to the target chemical data. If yes, the non-limiting method 900 can proceed to step 908. If not, the non-limiting method 900 can proceed back to step 902 and/or 904.

At 908, the non-limiting method 900 can comprise generating, by the system (e.g., generating component 212), the target neutral loss data, based on target spectral data corresponding to the target molecular data and corresponding to the target sample, in a non-encoded format.

At 910, the non-limiting method 900 can comprise comparing, by the system, (e.g., comparing component 214 and/or model 222), encoded known molecular data, corresponding to the known spectral data, and the encoded target molecular data.

At 912, the non-limiting method 900 can comprise generating, by the system, (e.g., ranking component 216 and/or model 222), rankings for a set of one or more known samples, including the known sample, based on a first level of similarity of encoded known spectral data, corresponding to the one or more known samples, to the encoded target molecular data.

At 914, the non-limiting method 900 can comprise comparing, by the system, (e.g., comparing component 214 and/or model 222), the known neutral loss data and target neutral loss data, for the target sample, resulting in a set of one or more possible matches, including a predicted match, of one or more known samples corresponding to the target sample.

At 916, the non-limiting method 900 can comprise executing, by the system (e.g., comparing component 214 and/or model 222), the comparing of the known neutral loss data and target neutral loss data wherein the known neutral loss data comprises a neutral loss represented by, but not defined by a spectrum corresponding to, the known spectral data.

At 918, the non-limiting method 900 can comprise generating, by the system (e.g., ranking component 216 and/or model 222), re-rankings of the set of one or more known samples, including the known sample, based on a second level of similarity of known neutral loss data, including the known neutral loss data, corresponding to the set of one or more known samples, to target neutral loss data corresponding to the target sample.

In one or more embodiments, re-ranking can be performed without the ranking.

In one or more embodiments, comparing, ranking and re-ranking can be performed in any suitable order and/or at least partially at a same time as one another.

At 920, the non-limiting method 900 can comprise applying, by the system (e.g., ranking component 216 and/or model 222), the second level of similarity to neutral loss data, of the known neutral loss data, that corresponds to known bits, of the encoded known molecular data, that match to target bits of the encoded target molecular data.

At 922, the non-limiting method 900 can comprise generating, by the system (e.g., weighting component 218 and/or model 222), a weight for a data aspect corresponding to the known sample, wherein the weight is generated based on an aggregated similarity between the encoded known molecular data and the encoded target molecular data, and between target neutral loss data corresponding to the target sample and the known neutral loss data.

In one or more embodiments, weighting can result from the ranking and re-ranking, just from the re-ranking, and/or can be in place of the ranking.

In one or more embodiments, comparing, ranking, re-ranking and weighting can be performed in any suitable order and/or at least partially at a same time as one another.

At 924, the non-limiting method 900 can comprise generating, by the system (e.g., matching component 220 and/or model 222), a predicted match of the encoded target molecular data, also in the vectorized format, to known neutral loss data for a known sample, the known neutral loss data defining a delta mass-to-charge ratio between spectral values of known spectral data corresponding to the known sample.

At 926, the non-limiting method 900 can comprise matching, by the system (e.g., matching component 220 and/or model 222), the known neutral loss data to a bit of the plurality of bits.

At 928, the non-limiting method 900 can comprise generating, by the system (e.g., notifying component 224), report data (e.g., notification 290) comprising cause data linking a structural feature of the target sample to specified neutral loss data, of the known neutral loss data, corresponding to at least one or more bits of the encoded known molecular data.

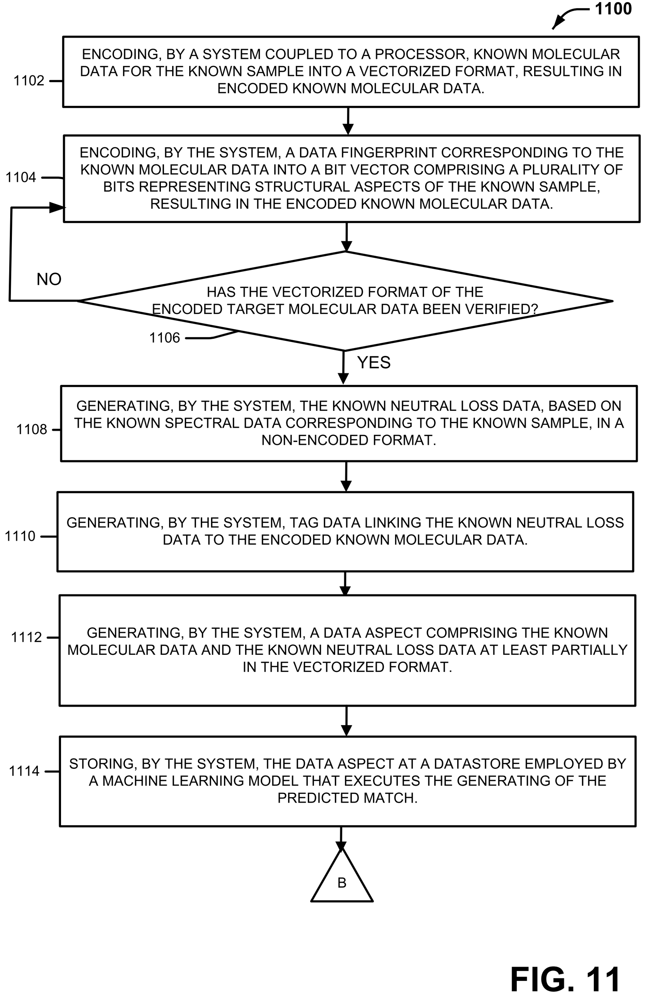

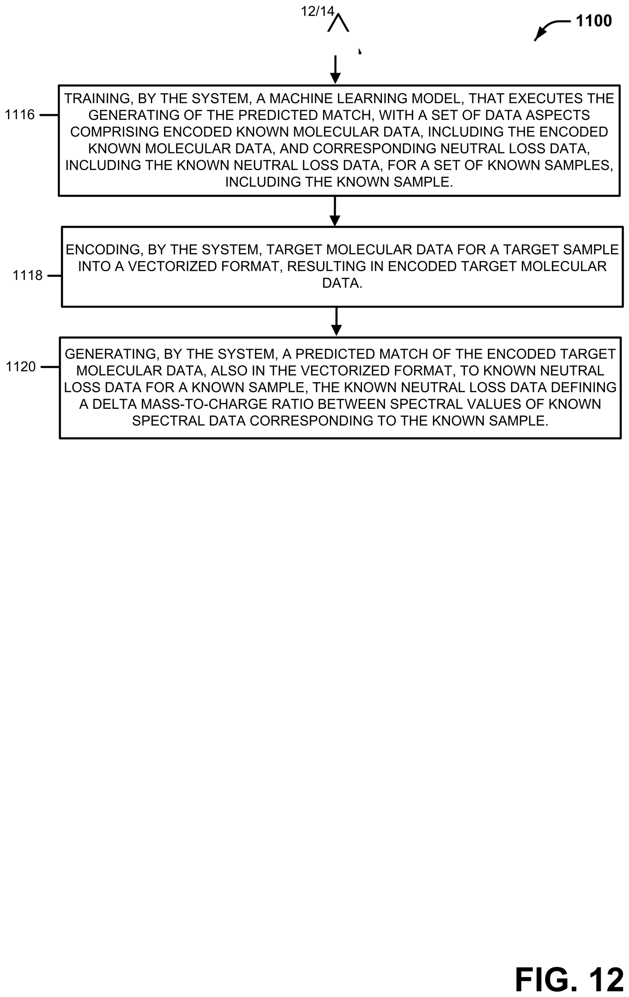

As another summary of the above-described components and/or functions thereof, referring next to FIGS. 11 and 12, illustrated is a flow diagram of an example, non-limiting method 1100 that can facilitate a process for chemical data comparison and target data annotation, in accordance with one or more example embodiments described herein, such as the non-limiting system 200 of FIG. 2. While the non-limiting method 1100 is described relative to the non-limiting system 200 of FIG. 2, the non-limiting method 1100 can be applicable also to other systems described herein, such as the non-limiting system 100 of FIG. 1. Repetitive description of like elements and/or processes employed in respective embodiments is omitted for sake of brevity.

At 1102, the non-limiting method 1100 can comprise encoding, by a system (e.g., encoding component 210), known molecular data for the known sample into a vectorized format, resulting in encoded known molecular data.

At 1104, the non-limiting method 1100 can comprise encoding, by the system (e.g., encoding component 210), a data fingerprint corresponding to the known molecular data into a bit vector comprising a plurality of bits representing structural aspects of the known sample, resulting in the encoded known molecular data.

At 1106, the non-limiting method 1100 can comprise determining, by the system (e.g., encoding component 210 and/or processor 206), whether the vectorized format of the encoded target molecular data has been verified, such as compared to a vectorized format employed for known chemical data to be employed for comparison to the target chemical data. If yes, the non-limiting method 1100 can proceed to step 1108. If not, the non-limiting method 1100 can proceed back to step 1102 and/or 1104.

At 1108, the non-limiting method 1100 can comprise generating, by the system (e.g., generating component 212), the known neutral loss data, based on the known spectral data corresponding to the known sample, in a non-encoded format.

At 1110, the non-limiting method 1100 can comprise generating, by the system, (e.g., generating component 212), tag data linking the known neutral loss data to the encoded known molecular data.

At 1112, the non-limiting method 1100 can comprise generating, by the system, (e.g., generating component 212), a data aspect comprising the known molecular data and the known neutral loss data at least partially in the vectorized format.

At 1114, the non-limiting method 1100 can comprise storing, by the system, (e.g., generating component 212 and/or training component 226), the data aspect at a datastore employed by a machine learning model that executes the generating of the predicted match.

At 1116, the non-limiting method 1100 can comprise training, by the system (e.g., training component 226), a machine learning model, that executes the generating of the predicted match, with a set of data aspects comprising encoded known molecular data, including the encoded known molecular data, and corresponding neutral loss data, including the known neutral loss data, for a set of known samples, including the known sample.

At 1118, the non-limiting method 1100 can comprise encoding, by the system (e.g., encoding component 210), target molecular data for a target sample into a vectorized format, resulting in encoded target molecular data.

At 1120, the non-limiting method 1100 can comprise generating, by the system (e.g., matching component 220 and/or model 222), a predicted match of the encoded target molecular data, also in the vectorized format, to known neutral loss data for a known sample, the known neutral loss data defining a delta mass-to-charge ratio between spectral values of known spectral data corresponding to the known sample. Additional Summary

For simplicity of explanation, the computer-implemented and non-computer-implemented methodologies provided herein are depicted and/or described as a series of acts. It is to be understood that the subject innovation is not limited by the acts illustrated and/or by the order of acts, for example acts can occur in one or more orders and/or concurrently, and with other acts not presented and described herein. Furthermore, not all illustrated acts can be utilized to implement the computer-implemented and non-computer-implemented methodologies in accordance with the described subject matter. In addition, the computer-implemented and non-computer-implemented methodologies could alternatively be represented as a series of interrelated states via a state diagram or events. Additionally, the computer-implemented methodologies described hereinafter and throughout this specification are capable of being stored on an article of manufacture for transporting and transferring the computer-implemented methodologies to computers. The term article of manufacture, as used herein, is intended to encompass a computer program accessible from any computer-readable device or storage media.

The systems and/or devices have been (and/or will be further) described herein with respect to interaction between one or more components. Such systems and/or components can include those components or sub-components specified therein, one or more of the specified components and/or sub-components, and/or additional components. Sub-components can be implemented as components communicatively coupled to other components rather than included within parent components. One or more components and/or sub-components can be combined into a single component providing aggregate functionality. The components can interact with one or more other components not specifically described herein for the sake of brevity, but known by those of skill in the art.

In summary, embodiments described herein relate to target sample data annotation. A system can comprise a memory that stores, and a processor that executes, computer executable components. The computer executable components can comprise an encoding component 110, 210 that encodes target molecular data 126, 610 for a target sample 124, 602 into a vectorized format 128, 626, resulting in encoded target molecular data 130,628, and a matching component 120, 220 that generates a predicted match 170, 270 of the encoded target molecular data 126, 610 to known neutral loss data 136, 308 for a known sample 122, 302, the known neutral loss data 136, 308 defining a delta mass-to-charge ratio 138, 316 between spectral values 134, 312 of known spectral data 132, 306 corresponding to the known sample 122, 302.

The one or more example embodiments disclosed herein can be applied on a plug-and-play basis to a measurement device, plural measurement devices, a same measurement device using plural exchangeable components (e.g., columns), etc. for comparison of output data relative to unknown, known and/or standard data. The frameworks described herein can be performed in a time efficient and at least partially automatic manner, thereby reducing labor processes, increasing accuracy, and providing automatic reasoning for predictions made. In one or more cases, identification data obtained from use of the one or more example embodiments can be employed to construct a database of known molecular, neutral loss, and/or spectral data.

Accordingly, the one or more example embodiments described herein can be implemented within, in connection with and/or coupled to a scientific measurement device, such as a spectrometry device.

Indeed, in view of the one or more example embodiments described herein, a practical application of the one or more systems, computer-implemented methods and/or computer program products described herein can be an ability to generate inferences and/or neutral loss data corresponding to a target sample that would not otherwise be available by merely comparing molecular data and/or neutral loss data for the target sample to a library of known molecular data and/or known neutral loss data. That is, based on spectral data defining a spectrum, one or more neutral losses can be exhibited, while one or more other neutral losses can be non-exhibited. That is, such non-exhibited neutral losses can fail to appear during fragmentation such as due to a chemical structure (e.g., chemical bond type), chemical property, fragmentation energy not being reached, etc. Put another way, the non-exhibited neutral loss can correspond to an ion that has not fragmented from a target sample due to a same and/or different reason (e.g., chemical structure, chemical property, fragmentation energy).

That is, as compared to existing frameworks that cannot provide this ability, the one or more example embodiments described herein can be employed to leverage information related to one or more compounds different from a target compound for which annotation is desired. For example, a prediction regarding identification of a neutral loss (exhibited or not exhibited in spectral data), a prediction regarding identification of an ion based on a chemical structure (e.g., chemical bond type), and/or a prediction regarding identification of a target compound, without being limited thereto, can be made based on molecular structure data, neutral loss data and/or spectral data, without being limited thereto, that corresponds to a known compound different from the target compound. Such known compound can be of a same family, chemical category type, etc., and/or can have one or more structural features, ions and/or neutral losses in common with the target compound, for example. This prediction can be accomplished employing a database of hundreds, thousands, tens of thousands, or more sets of chromatography data, labeled peaks, etc., without being limited thereto.

In view of the foregoing advantages, benefits and/or features are useful and practical applications of computers, thus providing enhanced (e.g., improved and/or optimized) spectrometry data analysis. Overall, such computerized tools can constitute a concrete and tangible technical improvement in the fields of material analysis, and more particularly in analysis of scientific measurement device output, such as including, but not limited to, the field of spectrometry.

Furthermore, one or more example embodiments described herein can be employed in a real-world system based on the disclosed teachings. For example, one or more embodiments can employ one or more such data aspects, e.g., comprising molecular structure data, neutral loss data and/or spectral data, to compare ion identifications, compound molecular structures, spectral peak values, neutral loss values (e.g., gaps between spectral peaks), etc. of one or more target compounds and/or known compounds. Such comparison can be employed to annotate unknown and/or target compound data and/or to generate a data library of data aspects. Such comparison can be accomplished employing a database of hundreds, thousands, tens of thousands, or more sets of data aspects, without being limited thereto.

Moreover, based on the comparison, a more comprehensive understanding of the target spectral data can be obtained, as compared to existing frameworks. For example, one or more structural and/or neutral loss characteristics can be predicted by use of a model, such as an artificial intelligence (AI) model or machine learning (ML) model employing the database and having learned correspondences among molecular structure data, neutral loss data and/or spectral data for the data aspects comprised by the database. One or more resultant identified peaks, characteristics, ions, neutral losses, etc., relating to a known or unknown compound can be predicted, with one or more outputs being predicted per such result, such as in a ranked and/or weighted format. In one or more cases, ranked and/or weighted data can be accompanied by and/or provided separately from one or more correspondence-based (e.g., correspondences among the molecular structure data, neutral loss data and/or spectral data) reasons for the ranked and/or weighted data. This can allow for an understanding of target molecular structure data, neutral loss data and/or spectral data and its causes and/or the reasoning behind any one or more identifications provided by the model. Put briefly, the embodiments disclosed herein thus can provide improvements to scientific instrument technology (e.g., improvements in the computer technology supporting such scientific instruments, among other improvements).

Moreover, the one or more example embodiments described herein can achieve a level of scale of operation. For example, spectrometry data (e.g., target sample data 246) corresponding to two or more compounds (e.g., target samples 602) can be evaluated at least partially in parallel with one another relative to same and/or different systems, measurement devices, databases of known chemical data, etc.

The systems and/or devices have been (and/or will be further) described herein with respect to interaction between one or more components. Such systems and/or components can include those components or sub-components specified therein, one or more of the specified components and/or sub-components, and/or additional components. Sub-components can be implemented as components communicatively coupled to other components rather than included within parent components. One or more components and/or sub-components can be combined into a single component providing aggregate functionality. For example, as noted above, in one or more embodiments, the model 222 can comprise, and/or perform one or more functions described as being comprised by, one or more of the matching component 220, weighting component 218, ranking component 216 and/or comparing component 214. The components can interact with one or more other components not specifically described herein for the sake of brevity, but known by those of skill in the art.

One or more example embodiments described herein can be, in one or more cases, inherently and/or inextricably tied to computer technology and cannot be implemented outside of a computing environment. For example, one or more processes performed by one or more example embodiments described herein can more efficiently, and even more feasibly, provide program and/or program instruction execution, such as relative to measurement device output comparison (e.g., measurement device use for material analysis), as compared to existing systems and/or techniques using molecular network generation and/or visualization. Systems, computer-implemented methods and/or computer program products providing performance of these processes are of great utility in the fields of material analysis and cannot be equally practicably implemented in a sensible way outside of a computing environment.

One or more example embodiments described herein can employ hardware and/or software to solve problems that are highly technical, that are not abstract, and that cannot be performed as a set of mental acts by a human. For example, a human, or even thousands of humans, cannot efficiently, accurately and/or effectively analyze computer data/metadata (e.g., defining spectrometry data) defining fragmented ion mass to charge ratios, intensities, inferenced neutral losses, etc. at one or more measurement devices, and/or generate a digital display visual of quantified similarities and/or differences between chemical datasets, as the one or more example embodiments described herein can provide this process. Moreover, neither can the human mind nor a human with pen and paper conduct one or more of these processes, as conducted by one or more example embodiments described herein.

In one or more example embodiments, one or more of the processes described herein can be performed by one or more specialized computers (e.g., a specialized processing unit, a specialized classical computer, a specialized quantum computer, a specialized hybrid classical/quantum system and/or another type of specialized computer) to execute defined tasks related to the one or more technologies describe above. One or more example embodiments described herein and/or components thereof can be employed to solve new problems that arise through advancements in technologies mentioned above, employment of quantum computing systems, cloud computing systems, computer architecture and/or another technology.

One or more example embodiments described herein can be fully operational towards performing one or more other functions (e.g., fully powered on, fully executed and/or another function) while also performing one or more of the one or more operations described herein.

To provide additional summary, a listing of embodiments and features thereof is next provided.

A system, comprising: a memory that stores computer executable components; and a processor that executes the computer executable components stored in the memory, wherein the computer executable components comprise: an encoding component that encodes target molecular data for a target sample into a vectorized format, resulting in encoded target molecular data; and a matching component that generates a predicted match of the encoded target molecular data to known neutral loss data for a known sample, the known neutral loss data defining a delta mass-to-charge ratio between spectral values of known spectral data corresponding to the known sample.

The system of the preceding paragraph, wherein the known neutral loss data comprises a neutral loss represented by, but not defined by a spectrum corresponding to, the known spectral data.

The system of any preceding paragraph, wherein the encoding component encodes a data fingerprint corresponding to the target molecular data into a bit vector comprising a plurality of bits representing structural aspects of the target sample, resulting in the encoded target molecular data, and wherein the matching component matches the known neutral loss data to a bit of the plurality of bits.

The system of any preceding paragraph, wherein the computer executable components further comprise: a comparing component that compares encoded known molecular data, corresponding to the known spectral data, to the encoded target molecular data, and that compares the known neutral loss data and target neutral loss data, for the target sample, resulting in a set of one or more possible matches, including the predicted match, of one or more known samples corresponding to the target sample.

The system of any preceding paragraph, wherein the computer executable components further comprise: a ranking component that generates rankings for a set of one or more known samples, including the known sample, based on a first level of similarity of encoded known molecular data, corresponding to the one or more known samples, to the encoded target molecular data.

The system of any preceding paragraph, wherein the ranking component further generates re-rankings of the set of one or more known samples, including the known sample, based on a second level of similarity of known neutral loss data, including the known neutral loss data, corresponding to the set of one or more known samples, to target neutral loss data corresponding to the target sample.

The system of any preceding paragraph, wherein the computer executable components further comprise: a generating component that generates the target neutral loss data, based on target spectral data corresponding to the target molecular data and corresponding to the target sample, in a non-encoded format.

The system of any preceding paragraph, wherein the second level of similarity is applied to neutral loss data, of the known neutral loss data, that corresponds to known bits, of the encoded known molecular data, that match to target bits of the encoded target molecular data.

The system of any preceding paragraph, wherein the computer executable components further comprise: a weighting component that generates a weight for a data aspect corresponding to the known sample, wherein the weight is generated based on an aggregated similarity between the encoded known molecular data and the encoded target molecular data, and between target neutral loss data corresponding to the target sample and the known neutral loss data.

The system of any preceding paragraph, wherein the computer executable components further comprise: a notifying component that generates report data comprising cause data linking a structural feature of the target sample to specified neutral loss data, of the known neutral loss data, corresponding to at least one or more bits of the encoded known molecular data.

A computer-implemented method, comprising: encoding, by a system operatively coupled to a processor, target molecular data for a target sample into a vectorized format, resulting in encoded target molecular data; and generating, by the system, a predicted match of the encoded target molecular data to known neutral loss data for a known sample, the known neutral loss data defining a delta mass-to-charge ratio between spectral values of known spectral data corresponding to the known sample.

The computer-implemented method of any preceding paragraph, wherein the known neutral loss data comprises a neutral loss represented by, but not defined by a spectrum corresponding to, the known spectral data.

The computer-implemented method of any preceding paragraph, further comprising: encoding, by the system, known molecular data for the known sample into a vectorized format, resulting in encoded known molecular data; and generating, by the system, the known neutral loss data, based on the known spectral data corresponding to the known sample, in a non-encoded format.

The computer-implemented method of any preceding paragraph, further comprising: encoding, by the system, a data fingerprint corresponding to the known molecular data into a bit vector comprising a plurality of bits representing structural aspects of the known sample, resulting in the encoded known molecular data.

The computer-implemented method of any preceding paragraph, further comprising: generating, by the system, tag data linking the known neutral loss data to the encoded known molecular data.

The computer-implemented method of any preceding paragraph, further comprising: generating, by the system, a data aspect comprising the known molecular data and the known neutral loss data at least partially in the vectorized format; and storing, by the system, the data aspect at a datastore employed by a machine learning model that executes the generating of the predicted match.

The computer-implemented method of any preceding paragraph, further comprising: training, by the system, a machine learning model, that executes the generating of the predicted match, with a set of data aspects comprising encoded known molecular data, including the encoded known molecular data, and corresponding neutral loss data, including the known neutral loss data, for a set of known samples, including the known sample.

A computer program product facilitating a process for target sample annotation, the computer program product comprising a computer readable storage medium having program instructions embodied therewith, and the program instructions executable by a processor to cause the processor to: encode, by the processor, target molecular data for a target sample into a vectorized format, resulting in encoded target molecular data; and generate, by the processor, a predicted match of the encoded target molecular data to known neutral loss data for a known sample, the known neutral loss data defining a delta mass-to-charge ratio between spectral values of known spectral data corresponding to the known sample.

The computer program product of any preceding paragraph, wherein the known neutral loss data comprises a neutral loss represented by, but not defined by a spectrum corresponding to, the known spectral data.

The computer program product of any preceding paragraph, wherein the program instructions are further executable by the processor to cause the processor to: encode, by the processor, a data fingerprint corresponding to the target molecular data into a bit vector comprising a plurality of bits representing structural aspects of the target sample, resulting in the encoded target molecular data; and match, by the processor, the known neutral loss data to a bit of the plurality of bits.

### Example Operating Environment

FIG. 13 is a schematic block diagram of an operating environment 1300 with which the described subject matter can interact. The operating environment 1300 comprises one or more remote component(s) 1310. The remote component(s) 1310 can be hardware and/or software (e.g., threads, processes, computing devices). In one or more example embodiments, remote component(s) 1310 can be a distributed computer system, connected to a local automatic scaling component and/or programs that use the resources of a distributed computer system, via communication framework 1340. Communication framework 1340 can comprise wired network devices, wireless network devices, mobile devices, wearable devices, radio access network devices, gateway devices, femtocell devices, servers, etc.

The operating environment 1300 also comprises one or more local component(s) 1320. The local component(s) 1320 can be hardware and/or software (e.g., threads, processes, computing devices). In one or more example embodiments, local component(s) 1320 can comprise an automatic scaling component and/or programs that communicate/use the remote resources 1310 and 1320, etc., connected to a remotely located distributed computing system via communication framework 1340.

One possible communication between a remote component(s) 1310 and a local component(s) 1320 can be in the form of a data packet adapted to be transmitted between two or more computer processes. Another possible communication between a remote component(s) 1310 and a local component(s) 1320 can be in the form of circuit-switched data adapted to be transmitted between two or more computer processes in radio time slots. The operating environment 1300 comprises a communication framework 1340 that can be employed to facilitate communications between the remote component(s) 1310 and the local component(s) 1320, and can comprise an air interface, e.g., interface of a UMTS network, via an LTE network, etc. Remote component(s) 1310 can be operably connected to one or more remote data store(s) 1350, such as a hard drive, solid state drive, subscriber identity module (SIM) card, electronic SIM (eSIM), device memory, etc., that can be employed to store information on the remote component(s) 1310 side of communication framework 1340. Similarly, local component(s) 1320 can be operably connected to one or more local data store(s) 1330, that can be employed to store information on the local component(s) 1320 side of communication framework 1340.

### Example Computing Environment

In order to provide additional context for various embodiments described herein, FIG. 14 and the following discussion are intended to provide a brief, general description of a suitable computing environment 1400 in which the various embodiments of the embodiment described herein can be implemented. While the embodiments have been described above in the general context of computer-executable instructions that can run on one or more computers, those skilled in the art will recognize that the embodiments can be also implemented in combination with other program modules and/or as a combination of hardware and software.

Generally, program modules include routines, programs, components, data structures, etc., that perform tasks or implement abstract data types. Moreover, the methods can be practiced with other computer system configurations, including single-processor or multiprocessor computer systems, minicomputers, mainframe computers, Internet of Things (IoT) devices, distributed computing systems, as well as personal computers, hand-held computing devices, microprocessor-based or programmable consumer electronics, and the like, each of which can be operatively coupled to one or more associated devices.

The illustrated embodiments of the embodiments herein can also be practiced in distributed computing environments where certain tasks are performed by remote processing devices that are linked through a communications network. In a distributed computing environment, program modules can be located in both local and remote memory storage devices.

Computing devices typically include a variety of media, which can include computer-readable storage media, machine-readable storage media, and/or communications media, which two terms are used herein differently from one another as follows. Computer-readable storage media or machine-readable storage media can be any available storage media that can be accessed by the computer and includes both volatile and nonvolatile media, removable and non-removable media. By way of example, and not limitation, computer-readable storage media or machine-readable storage media can be implemented in connection with any method or technology for storage of information such as computer-readable or machine-readable instructions, program modules, structured data, or unstructured data.

Computer-readable storage media can include, but are not limited to, random access memory (RAM), read only memory (ROM), electrically erasable programmable read only memory (EEPROM), flash memory or other memory technology, compact disk read only memory (CD ROM), digital versatile disk (DVD), Blu-ray disc (BD) or other optical disk storage, magnetic cassettes, magnetic tape, magnetic disk storage or other magnetic storage devices, solid state drives or other solid state storage devices, or other tangible and/or non-transitory media which can be used to store desired information. In this regard, the terms "tangible" or "non-transitory" herein as applied to storage, memory, or computer-readable media, exclude only propagating transitory signals per se as modifiers and do not relinquish rights to all standard storage, memory or computer-readable media that are not only propagating transitory signals per se.

Computer-readable storage media can be accessed by one or more local or remote computing devices, e.g., via access requests, queries, or other data retrieval protocols, for a variety of operations with respect to the information stored by the medium.

Communications media typically embody computer-readable instructions, data structures, program modules or other structured or unstructured data in a data signal such as a modulated data signal, e.g., a carrier wave or other transport mechanism, and includes any information delivery or transport media. The term "modulated data signal" or signals refers to a signal that has one or more of its characteristics set or changed in such a manner as to encode information in one or more signals. By way of example, and not limitation, communication media include wired media, such as a wired network or direct-wired connection, and wireless media such as acoustic, RF, infrared and other wireless media.

Referring still to FIG. 14, the example computing environment 1400 which can implement one or more example embodiments described herein includes a computer 1402, the computer 1402 including a processing unit 1404, a system memory 1406 and a system bus 1408. The system bus 1408 couples system components including, but not limited to, the system memory 1406 to the processing unit 1404. The processing unit 1404 can be any of various commercially available processors. Dual microprocessors and other multi processor architectures can also be employed as the processing unit 1404.

The system bus 1408 can be any of several types of bus structure that can further interconnect to a memory bus (with or without a memory controller), a peripheral bus, and a local bus using any of a variety of commercially available bus architectures. The system memory 1406 includes ROM 1410 and RAM 1412. A basic input/output system (BIOS) can be stored in a non-volatile memory such as ROM, erasable programmable read only memory (EPROM), EEPROM, which BIOS contains the basic routines that help to transfer information between elements within the computer 1402, such as during startup. The RAM 1412 can also include a high-speed RAM such as static RAM for caching data.

The computer 1402 further includes an internal hard disk drive (HDD) 1414 (e.g., EIDE, SATA), and can include one or more external storage devices 1416 (e.g., a magnetic floppy disk drive (FDD) 1416, a memory stick or flash drive reader, a memory card reader, etc.). While the internal HDD 1414 is illustrated as located within the computer 1402, the internal HDD 1414 can also be configured for external use in a suitable chassis (not shown). Additionally, while not shown in computing environment 1400, a solid-state drive (SSD) could be used in addition to, or in place of, an HDD 1414.

Other internal or external storage can include at least one other storage device 1420 with storage media 1422 (e.g., a solid-state storage device, a nonvolatile memory device, and/or an optical disk drive that can read or write from removable media such as a CD-ROM disc, a DVD, a BD, etc.). The external storage 1416 can be facilitated by a network virtual machine. The HDD 1414, external storage device 1416 and storage device (e.g., drive) 1420 can be connected to the system bus 1408 by an HDD interface 1424, an external storage interface 1426 and a drive interface 1428, respectively.

The drives and their associated computer-readable storage media provide nonvolatile storage of data, data structures, computer-executable instructions, and so forth. For the computer 1402, the drives and storage media accommodate the storage of any data in a suitable digital format. Although the description of computer-readable storage media above refers to respective types of storage devices, other types of storage media which are readable by a computer, whether presently existing or developed in the future, could also be used in the example operating environment, and further, that any such storage media can contain computer-executable instructions for performing the methods described herein.

A number of program modules can be stored in the drives and RAM 1412, including an operating system 1430, one or more application programs 1432, other program modules 1434 and program data 1436. All or portions of the operating system, applications, modules, and/or data can also be cached in the RAM 1412. The systems and methods described herein can be implemented utilizing various commercially available operating systems or combinations of operating systems.

Computer 1402 can optionally comprise emulation technologies. For example, a hypervisor (not shown) or other intermediary can emulate a hardware environment for operating system 1430, and the emulated hardware can optionally be different from the hardware illustrated in FIG. 14. In such an embodiment, operating system 1430 can comprise one virtual machine (VM) of multiple VMs hosted at computer 1402. Furthermore, operating system 1430 can provide runtime environments, such as the Java runtime environment or the .NET framework, for applications 1432. Runtime environments are consistent execution environments that allow applications 1432 to run on any operating system that includes the runtime environment. Similarly, operating system 1430 can support containers, and applications 1432 can be in the form of containers, which are lightweight, standalone, executable packages of software that include, e.g., code, runtime, system tools, system libraries and settings for an application.

Further, computer 1402 can be enabled with a security module, such as a trusted processing module (TPM). For instance, with a TPM, boot components hash next in time boot components and wait for a match of results to secured values, before loading a next boot component. This process can take place at any layer in the code execution stack of computer 1402, e.g., applied at the application execution level or at the operating system (OS) kernel level, thereby enabling security at any level of code execution.

A user entity can enter commands and information into the computer 1402 through one or more wired/wireless input devices, e.g., a keyboard 1438, a touch screen 1440, and a pointing device, such as a mouse 1442. Other input devices (not shown) can include a microphone, an infrared (IR) remote control, a radio frequency (RF) remote control, or other remote control, a joystick, a virtual reality controller and/or virtual reality headset, a game pad, a stylus pen, an image input device, e.g., camera, a gesture sensor input device, a vision movement sensor input device, an emotion or facial detection device, a biometric input device, e.g., fingerprint or iris scanner, or the like. These and other input devices are often connected to the processing unit 1404 through an input device interface 1444 that can be coupled to the system bus 1408, but can be connected by other interfaces, such as a parallel port, an IEEE 1394 serial port, a game port, a USB port, an IR interface, a BLUETOOTH^{®} interface, etc.

A monitor 1446 or other type of display device can also be connected to the system bus 1408 via an interface, such as a video adapter 1448. In addition to the monitor 1446, a computer typically includes other peripheral output devices (not shown), such as speakers, printers, etc.

The computer 1402 can operate in a networked environment using logical connections via wired and/or wireless communications to one or more remote computers, such as a remote computer 1450. The remote computer 1450 can be a workstation, a server computer, a router, a personal computer, portable computer, microprocessor-based entertainment appliance, a peer device or other common network node, and typically includes many or all of the elements described relative to the computer 1402, although, for purposes of brevity, only a memory/storage device 1452 is illustrated. The logical connections depicted include wired/wireless connectivity to a local area network (LAN) 1454 and/or larger networks, e.g., a wide area network (WAN) 1456. Such LAN and WAN networking environments are commonplace in offices and companies, and facilitate enterprise-wide computer networks, such as intranets, all of which can connect to a global communications network, e.g., the Internet.

When used in a LAN networking environment, the computer 1402 can be connected to the local network 1454 through a wired and/or wireless communication network interface or adapter 1458. The adapter 1458 can facilitate wired or wireless communication to the LAN 1454, which can also include a wireless access point (AP) disposed thereon for communicating with the adapter 1458 in a wireless mode.

When used in a WAN networking environment, the computer 1402 can include a modem 1460 or can be connected to a communications server on the WAN 1456 via other means for establishing communications over the WAN 1456, such as by way of the Internet. The modem 1460, which can be internal or external and a wired or wireless device, can be connected to the system bus 1408 via the input device interface 1444. In a networked environment, program modules depicted relative to the computer 1402 or portions thereof, can be stored in the remote memory/storage device 1452. The network connections shown are example and other means of establishing a communications link between the computers can be used.

When used in either a LAN or WAN networking environment, the computer 1402 can access cloud storage systems or other network-based storage systems in addition to, or in place of, external storage devices 1416 as described above. Generally, a connection between the computer 1402 and a cloud storage system can be established over a LAN 1454 or WAN 1456 e.g., by the adapter 1458 or modem 1460, respectively. Upon connecting the computer 1402 to an associated cloud storage system, the external storage interface 1426 can, with the aid of the adapter 1458 and/or modem 1460, manage storage provided by the cloud storage system as it would other types of external storage. For instance, the external storage interface 1426 can be configured to provide access to cloud storage sources as if those sources were physically connected to the computer 1402.

The computer 1402 can be operable to communicate with any wireless devices or entities operatively disposed in wireless communication, e.g., a printer, scanner, desktop and/or portable computer, portable data assistant, communications satellite, any piece of equipment or location associated with a wirelessly detectable tag (e.g., a kiosk, news stand, store shelf, etc.), and telephone. This can include Wireless Fidelity (Wi-Fi) and BLUETOOTH^{®} wireless technologies. Thus, the communication can be a defined structure as with an existing network or simply an ad hoc communication between at least two devices.

### Additional Information

The embodiments described herein can be directed to one or more of a system, a method, an apparatus and/or a computer program product at any possible technical detail level of integration. The computer program product can include a computer readable storage medium (or media) having computer readable program instructions thereon for causing a processor to carry out aspects of the one or more example embodiments described herein. The computer readable storage medium can be a tangible device that can retain and store instructions for use by an instruction execution device. The computer readable storage medium can be, for example, but is not limited to, an electronic storage device, a magnetic storage device, an optical storage device, an electromagnetic storage device, a superconducting storage device and/or any suitable combination of the foregoing. A non-exhaustive list of more specific examples of the computer readable storage medium can also include the following: a portable computer diskette, a hard disk, a random access memory (RAM), a read-only memory (ROM), an erasable programmable read-only memory (EPROM or Flash memory), a static random access memory (SRAM), a portable compact disc read-only memory (CD-ROM), a digital versatile disk (DVD), a memory stick, a floppy disk, a mechanically encoded device such as punch-cards or raised structures in a groove having instructions recorded thereon and/or any suitable combination of the foregoing. A computer readable storage medium, as used herein, is not to be construed as being transitory signals per se, such as radio waves and/or other freely propagating electromagnetic waves, electromagnetic waves propagating through a waveguide and/or other transmission media (e.g., light pulses passing through a fiber-optic cable), and/or electrical signals transmitted through a wire.

Computer readable program instructions described herein can be downloaded to respective computing/processing devices from a computer readable storage medium and/or to an external computer or external storage device via a network, for example, the Internet, a local area network, a wide area network and/or a wireless network. The network can comprise copper transmission cables, optical transmission fibers, wireless transmission, routers, firewalls, switches, gateway computers and/or edge servers. A network adapter card or network interface in each computing/processing device receives computer readable program instructions from the network and forwards the computer readable program instructions for storage in a computer readable storage medium within the respective computing/processing device. Computer readable program instructions for carrying out operations of the one or more example embodiments described herein can be assembler instructions, instruction-set-architecture (ISA) instructions, machine instructions, machine dependent instructions, microcode, firmware instructions, state-setting data, configuration data for integrated circuitry, and/or source code and/or object code written in any combination of one or more programming languages, including an object oriented programming language such as Smalltalk, C++ or the like, and/or procedural programming languages, such as the "C" programming language and/or similar programming languages. The computer readable program instructions can execute entirely on a computer, partly on a computer, as a stand-alone software package, partly on a computer and/or partly on a remote computer or entirely on the remote computer and/or server. In the latter scenario, the remote computer can be connected to a computer through any type of network, including a local area network (LAN) and/or a wide area network (WAN), and/or the connection can be made to an external computer (for example, through the Internet using an Internet Service Provider). In one or more example embodiments, electronic circuitry including, for example, programmable logic circuitry, field-programmable gate arrays (FPGA) and/or programmable logic arrays (PLA) can execute the computer readable program instructions by utilizing state information of the computer readable program instructions to personalize the electronic circuitry, in order to perform aspects of the one or more example embodiments described herein.

Aspects of the one or more example embodiments described herein are described with reference to flowchart illustrations and/or block diagrams of methods, apparatus (systems), and computer program products according to one or more example embodiments described herein. It will be understood that each block of the flowchart illustrations and/or block diagrams, and combinations of blocks in the flowchart illustrations and/or block diagrams, can be implemented by computer readable program instructions. These computer readable program instructions can be provided to a processor of a general-purpose computer, special purpose computer and/or other programmable data processing apparatus to produce a machine, such that the instructions, which execute via the processor of the computer or other programmable data processing apparatus, can create means for implementing the functions/acts specified in the flowchart and/or block diagram block or blocks. These computer readable program instructions can also be stored in a computer readable storage medium that can direct a computer, a programmable data processing apparatus and/or other devices to function in a particular manner, such that the computer readable storage medium having instructions stored therein can comprise an article of manufacture including instructions which can implement aspects of the function/act specified in the flowchart and/or block diagram block or blocks. The computer readable program instructions can also be loaded onto a computer, other programmable data processing apparatus and/or other device to cause a series of operational acts to be performed on the computer, other programmable apparatus and/or other device to produce a computer implemented process, such that the instructions which execute on the computer, other programmable apparatus and/or other device implement the functions/acts specified in the flowchart and/or block diagram block or blocks.

The flowcharts and block diagrams in the figures illustrate the architecture, functionality and/or operation of possible implementations of systems, computer-implementable methods and/or computer program products according to one or more example embodiments described herein. In this regard, each block in the flowchart or block diagrams can represent a module, segment and/or portion of instructions, which comprises one or more executable instructions for implementing the specified logical function. In one or more alternative implementations, the functions noted in the blocks can occur out of the order noted in the Figures. For example, two blocks shown in succession can be executed substantially concurrently, and/or the blocks can sometimes be executed in the reverse order, depending upon the functionality involved. It will also be noted that each block of the block diagrams and/or flowchart illustration, and/or combinations of blocks in the block diagrams and/or flowchart illustration, can be implemented by special purpose hardware-based systems that can perform the specified functions and/or acts and/or carry out one or more combinations of special purpose hardware and/or computer instructions.

While the subject matter has been described above in the general context of computer-executable instructions of a computer program product that runs on a computer and/or computers, those skilled in the art will recognize that the one or more example embodiments herein also can be implemented at least partially in parallel with one or more other program modules. Generally, program modules include routines, programs, components and/or data structures that perform particular tasks and/or implement particular abstract data types. Moreover, the aforedescribed computer-implemented methods can be practiced with other computer system configurations, including single-processor and/or multiprocessor computer systems, mini-computing devices, mainframe computers, as well as computers, hand-held computing devices (e.g., PDA, phone), and/or microprocessor-based or programmable consumer and/or industrial electronics. The illustrated aspects can also be practiced in distributed computing environments in which tasks are performed by remote processing devices that are linked through a communications network. However, one or more, if not all aspects of the one or more example embodiments described herein can be practiced on stand-alone computers. In a distributed computing environment, program modules can be located in both local and remote memory storage devices.

As used in this application, the terms "component," "system," "platform" and/or "interface" can refer to and/or can include a computer-related entity or an entity related to an operational machine with one or more specific functionalities. The entities described herein can be either hardware, a combination of hardware and software, software, or software in execution. For example, a component can be, but is not limited to being, a process running on a processor, a processor, an object, an executable, a thread of execution, a program and/or a computer. By way of illustration, both an application running on a server and the server can be a component. One or more components can reside within a process and/or thread of execution and a component can be localized on one computer and/or distributed between two or more computers. In another example, respective components can execute from various computer readable media having various data structures stored thereon. The components can communicate via local and/or remote processes such as in accordance with a signal having one or more data packets (e.g., data from one component interacting with another component in a local system, distributed system and/or across a network such as the Internet with other systems via the signal). As another example, a component can be an apparatus with specific functionality provided by mechanical parts operated by electric or electronic circuitry, which is operated by a software and/or firmware application executed by a processor. In such a case, the processor can be internal and/or external to the apparatus and can execute at least a part of the software and/or firmware application. As yet another example, a component can be an apparatus that provides specific functionality through electronic components without mechanical parts, where the electronic components can include a processor and/or other means to execute software and/or firmware that confers at least in part the functionality of the electronic components. In an aspect, a component can emulate an electronic component via a virtual machine, e.g., within a cloud computing system.

In addition, the term "or" is intended to mean an inclusive "or" rather than an exclusive "or." That is, unless specified otherwise, or clear from context, "X employs A or B" is intended to mean any of the natural inclusive permutations. That is, if X employs A; X employs B; or X employs both A and B, then "X employs A or B" is satisfied under any of the foregoing instances. Moreover, articles "a" and "an" as used in the subject specification and annexed drawings should generally be construed to mean "one or more" unless specified otherwise or clear from context to be directed to a singular form. As used herein, the terms "example" and/or "exemplary" are utilized to mean serving as an example, instance, or illustration. For the avoidance of doubt, the subject matter described herein is not limited by such examples. In addition, any aspect or design described herein as an "example" and/or "exemplary" is not necessarily to be construed as preferred or advantageous over other aspects or designs, nor is it meant to preclude equivalent exemplary structures and techniques known to those of ordinary skill in the art.

As it is employed in the subject specification, the term "processor" can refer to substantially any computing processing unit and/or device comprising, but not limited to, single-core processors; single-processors with software multithread execution capability; multi-core processors; multi-core processors with software multithread execution capability; multi-core processors with hardware multithread technology; parallel platforms; and/or parallel platforms with distributed shared memory. Additionally, a processor can refer to an integrated circuit, an application specific integrated circuit (ASIC), a digital signal processor (DSP), a field programmable gate array (FPGA), a programmable logic controller (PLC), a complex programmable logic device (CPLD), a discrete gate or transistor logic, discrete hardware components, and/or any combination thereof designed to perform the functions described herein. Further, processors can exploit nano-scale architectures such as, but not limited to, molecular and quantum-dot based transistors, switches and/or gates, in order to optimize space usage and/or to enhance performance of related equipment. A processor can be implemented as a combination of computing processing units.

Herein, terms such as "store," "storage," "data store," data storage," "database," and substantially any other information storage component relevant to operation and functionality of a component are utilized to refer to "memory components," entities embodied in a "memory," or components comprising a memory. Memory and/or memory components described herein can be either volatile memory or nonvolatile memory or can include both volatile and nonvolatile memory. By way of illustration, and not limitation, nonvolatile memory can include read only memory (ROM), programmable ROM (PROM), electrically programmable ROM (EPROM), electrically erasable ROM (EEPROM), flash memory and/or nonvolatile random-access memory (RAM) (e.g., ferroelectric RAM (FeRAM). Volatile memory can include RAM, which can act as external cache memory, for example. By way of illustration and not limitation, RAM can be available in many forms such as synchronous RAM (SRAM), dynamic RAM (DRAM), synchronous DRAM (SDRAM), double data rate SDRAM (DDR SDRAM), enhanced SDRAM (ESDRAM), Synchlink DRAM (SLDRAM), direct Rambus RAM (DRRAM), direct Rambus dynamic RAM (DRDRAM) and/or Rambus dynamic RAM (RDRAM). Additionally, the described memory components of systems and/or computer-implemented methods herein are intended to include, without being limited to including, these and/or any other suitable types of memory.

What has been described above includes mere examples of systems and computer-implemented methods. It is, of course, not possible to describe every conceivable combination of components and/or computer-implemented methods for purposes of describing the one or more example embodiments, but one of ordinary skill in the art can recognize that many further combinations and/or permutations of the one or more example embodiments are possible. Furthermore, to the extent that the terms "includes," "has," "possesses," and the like are used in the detailed description, claims, appendices and/or drawings such terms are intended to be inclusive in a manner similar to the term "comprising" as "comprising" is interpreted when employed as a transitional word in a claim.

The descriptions of the various embodiments can use the phrases "an embodiment," "various embodiments," "one or more example embodiments" and/or "some embodiments," each of which can refer to one or more of the same or different embodiments.

The descriptions of the various embodiments have been presented for purposes of illustration but are not intended to be exhaustive or limited to the embodiments described herein. Many modifications and variations will be apparent to those of ordinary skill in the art without departing from the scope and spirit of the described embodiments. The terminology used herein was chosen to best explain the principles of the embodiments, the practical application and/or technical improvement over technologies found in the marketplace, and/or to enable others of ordinary skill in the art to understand the embodiments described herein.

Further aspects of the invention are set out in the following numbered clauses:
Clause 1. A system, comprising:
   a memory that stores computer executable components; and
   a processor that executes the computer executable components stored in the memory, wherein the computer executable components comprise:
   an encoding component that encodes target molecular data for a target sample into a vectorized format, resulting in encoded target molecular data; and
   a matching component that generates a predicted match of the encoded target molecular data to known neutral loss data for a known sample, the known neutral loss data defining a delta mass-to-charge ratio between spectral values of known spectral data corresponding to the known sample.
Clause 2. The system of clause 1, wherein the known neutral loss data comprises a neutral loss represented by, but not defined by a spectrum corresponding to, the known spectral data.
Clause 3. The system of clause 1, wherein the encoding component encodes a data fingerprint corresponding to the target molecular data into a bit vector comprising a plurality of bits representing structural aspects of the target sample, resulting in the encoded target molecular data, and wherein the matching component matches the known neutral loss data to a bit of the plurality of bits.
Clause 4. The system of clause 1, wherein the computer executable components further comprise: a comparing component that compares encoded known molecular data, corresponding to the known spectral data, to the encoded target molecular data, and that compares the known neutral loss data and target neutral loss data, for the target sample, resulting in a set of one or more possible matches, including the predicted match, of one or more known samples corresponding to the target sample.
Clause 5. The system of clause 1, wherein the computer executable components further comprise: a ranking component that generates rankings for a set of one or more known samples, including the known sample, based on a first level of similarity of encoded known molecular data, corresponding to the one or more known samples, to the encoded target molecular data.
Clause 6. The system of clause 5, wherein the ranking component further generates re-rankings of the set of one or more known samples, including the known sample, based on a second level of similarity of known neutral loss data, including the known neutral loss data, corresponding to the set of one or more known samples, to target neutral loss data corresponding to the target sample.
Clause 7. The system of clause 6, wherein the computer executable components further comprise: a generating component that generates the target neutral loss data, based on target spectral data corresponding to the target molecular data and corresponding to the target sample, in a non-encoded format.
Clause 8. The system of clause 6, wherein the second level of similarity is applied to neutral loss data, of the known neutral loss data, that corresponds to known bits, of the encoded known molecular data, that match to target bits of the encoded target molecular data.
Clause 9. The system of clause 1, wherein the computer executable components further comprise: a weighting component that generates a weight for a data aspect corresponding to the known sample, wherein the weight is generated based on an aggregated similarity between the encoded known molecular data and the encoded target molecular data, and between target neutral loss data corresponding to the target sample and the known neutral loss data.
Clause 10. The system of clause 1, wherein the computer executable components further comprise: a notifying component that generates report data comprising cause data linking a structural feature of the target sample to specified neutral loss data, of the known neutral loss data, corresponding to at least one or more bits of the encoded known molecular data.
Clause 11. A computer-implemented method, comprising: encoding, by a system operatively coupled to a processor, target molecular data for a target sample into a vectorized format, resulting in encoded target molecular data; and generating, by the system, a predicted match of the encoded target molecular data to known neutral loss data for a known sample, the known neutral loss data defining a delta mass-to-charge ratio between spectral values of known spectral data corresponding to the known sample.
Clause 12. The computer-implemented method of clause 11, wherein the known neutral loss data comprises a neutral loss represented by, but not defined by a spectrum corresponding to, the known spectral data.
Clause 13. The computer-implemented method of clause 11, further comprising: encoding, by the system, known molecular data for the known sample into a vectorized format, resulting in encoded known molecular data; and generating, by the system, the known neutral loss data, based on the known spectral data corresponding to the known sample, in a non-encoded format.
Clause 14. The computer-implemented method of clause 13, further comprising: encoding, by the system, a data fingerprint corresponding to the known molecular data into a bit vector comprising a plurality of bits representing structural aspects of the known sample, resulting in the encoded known molecular data.
Clause 15. The computer-implemented method of clause 13, further comprising: generating, by the system, tag data linking the known neutral loss data to the encoded known molecular data.
Clause 16. The computer-implemented method of clause 13, further comprising: generating, by the system, a data aspect comprising the known molecular data and the known neutral loss data at least partially in the vectorized format; and storing, by the system, the data aspect at a datastore employed by a machine learning model that executes the generating of the predicted match.
Clause 17. The computer-implemented method of clause 11, further comprising: training, by the system, a machine learning model, that executes the generating of the predicted match, with a set of data aspects comprising encoded known molecular data, including the encoded known molecular data, and corresponding neutral loss data, including the known neutral loss data, for a set of known samples, including the known sample.
Clause 18. A computer program product facilitating a process for target sample annotation, the computer program product comprising a computer readable storage medium having program instructions embodied therewith, and the program instructions executable by a processor to cause the processor to: encode, by the processor, target molecular data for a target sample into a vectorized format, resulting in encoded target molecular data; and generate, by the processor, a predicted match of the encoded target molecular data to known neutral loss data for a known sample, the known neutral loss data defining a delta mass-to-charge ratio between spectral values of known spectral data corresponding to the known sample.
Clause 19. The computer program product of clause 18, wherein the known neutral loss data comprises a neutral loss represented by, but not defined by a spectrum corresponding to, the known spectral data.
Clause 20. The computer program product of clause 18, wherein the program instructions are further executable by the processor to cause the processor to: encode, by the processor, a data fingerprint corresponding to the target molecular data into a bit vector comprising a plurality of bits representing structural aspects of the target sample, resulting in the encoded target molecular data; and match, by the processor, the known neutral loss data to a bit of the plurality of bits.

## Claims

1. A computer-implemented method, comprising:
encoding, by a system operatively coupled to a processor, target molecular data for a target sample into a vectorized format, resulting in encoded target molecular data; and
generating, by the system, a predicted match of the encoded target molecular data to known neutral loss data for a known sample, the known neutral loss data defining a delta mass-to-charge ratio between spectral values of known spectral data corresponding to the known sample.

2. The computer-implemented method of claim 1, wherein the known neutral loss data comprises a neutral loss represented by, but not defined by a spectrum corresponding to, the known spectral data.

3. The computer-implemented method of claim 1 or claim 2, further comprising:
encoding, by the system, known molecular data for the known sample into a vectorized format, resulting in encoded known molecular data; and
generating, by the system, the known neutral loss data, based on the known spectral data corresponding to the known sample, in a non-encoded format.

4. The computer-implemented method of claim 3, further comprising:
encoding, by the system, a data fingerprint corresponding to the known molecular data into a bit vector comprising a plurality of bits representing structural aspects of the known sample, resulting in the encoded known molecular data.

5. The computer-implemented method of claim 3, further comprising:
generating, by the system, tag data linking the known neutral loss data to the encoded known molecular data.

6. The computer-implemented method of claim 3, further comprising:
generating, by the system, a data aspect comprising the known molecular data and the known neutral loss data at least partially in the vectorized format; and
storing, by the system, the data aspect at a datastore employed by a machine learning model that executes the generating of the predicted match.

7. The computer-implemented method of any preceding claim, further comprising:
training, by the system, a machine learning model, that executes the generating of the predicted match, with a set of data aspects comprising encoded known molecular data, including the encoded known molecular data, and corresponding neutral loss data, including the known neutral loss data, for a set of known samples, including the known sample.

8. The method of claim 1, further comprising encoding a data fingerprint corresponding to the target molecular data into a bit vector comprising a plurality of bits representing structural aspects of the target sample, resulting in the encoded target molecular data, and
matching the known neutral loss data to a bit of the plurality of bits.

9. The method of claim 1, further comprising comparing encoded known molecular data, corresponding to the known spectral data, to the encoded target molecular data, and comparing the known neutral loss data and target neutral loss data, for the target sample, resulting in a set of one or more possible matches, including the predicted match, of one or more known samples corresponding to the target sample.

10. The method of claim 1, further comprising:
generating rankings for a set of one or more known samples, including the known sample, based on a first level of similarity of encoded known molecular data, corresponding to the one or more known samples, to the encoded target molecular data.

11. The method of claim 10, further comprising generating re-rankings of the set of one or more known samples, including the known sample, based on a second level of similarity of known neutral loss data, including the known neutral loss data, corresponding to the set of one or more known samples, to target neutral loss data corresponding to the target sample, and optionally generating the target neutral loss data, based on target spectral data corresponding to the target molecular data and corresponding to the target sample, in a non-encoded format, and further optionally wherein the second level of similarity is applied to neutral loss data, of the known neutral loss data, that corresponds to known bits, of the encoded known molecular data, that match to target bits of the encoded target molecular data.

12. The method of claim 1, further comprising generating a weight for a data aspect corresponding to the known sample, wherein the weight is generated based on an aggregated similarity between the encoded known molecular data and the encoded target molecular data, and between target neutral loss data corresponding to the target sample and the known neutral loss data.

13. The method of claim 1, further comprising generating report data comprising cause data linking a structural feature of the target sample to specified neutral loss data, of the known neutral loss data, corresponding to at least one or more bits of the encoded known molecular data.

14. A computer program for facilitating a process for target sample annotation, the computer program comprising program instructions executable by a processor to cause the processor to carry out the method steps of any of claims 1-13.

15. A system, comprising:
a memory that stores the computer program of claim 14; and
a processor that executes the computer program stored in the memory.
